# EUROPEAN PATENT APPLICATION

(11) **EP 4 454 702 A1**
(43) Date of publication of application: **30.10.2024**
(21) Application number: 24172128.1
(22) Date of filing: 24.04.2024
(51) Int. Cl.: A61N 5/10, A61B 6/40, A61B 6/42, A61B 6/00

(54) **FUNDAMENTALS OF SEMI-CONDUCTOR-BASED PHOTON COUNTING DETECTORS**

(30) Priority: 24.04.2023 US 202363497914 P
(71) Applicant: Accuray, Inc., Sunnyvale, CA 94089 (US)
(72) Inventor: Prekas, Georgios, Solon, OH, 44139 (US); Chappo, Marc, Elyria, OH, 44035 (US); Yu, Zhicong, Highland Hts., OH, 44143 (US); Gagnon, Daniel, Twinsburg, OH, 44087 (US)
(74) Representative: Grünecker Patent- und Rechtsanwälte PartG mbB

(57) **Abstract**

Disclosed herein is a treatment apparatus. The treatment apparatus includes a rotatable gantry system positioned at least partially around a patient support and a first source of radiation coupled to the rotatable gantry system. The first source of radiation is configured to emit imaging x-ray radiation after pausing for a time interval between periodic emissions. The apparatus further includes a radiation detector configured to receive x-ray radiation from the first radiation source and generate tomographic data from the received radiation. The radiation detector is subject to a transient effect caused by at least one of polarization and charge trapping. The time interval is sufficiently long for the transient effect to substantially dissipate.

## Description

### CROSS-REFERENCE TO RELATED APPLICATIONS

This application claims the priority of U.S. Provisional Application No. 63/497,914, filed April 24, 2023, entitled "Fundamentals of Semi-Conductor-Based Photon Counting Detectors" (Attorney Docket No. 38935.04116), the contents of which are fully incorporated herein by reference.

### FIELD OF THE INVENTION

This disclosure relates to intermittent or pulsed application of x-ray radiation and intermittent or pulsed bias voltage to improve imaging during radiotherapy. It includes applying pulsed x-ray radiation in computed tomography (CT) using energy-resolved photon-counting detection.

### BACKGROUND

The term "radiosurgery" refers to a procedure in which radiation is applied to a target region at doses sufficient to necrotize a pathology in fewer treatment sessions or fractions than with delivery of lower doses per fraction in a larger number of fractions. Radiosurgery is typically characterized, as distinguished from radiotherapy, by relatively high radiation doses per fraction (e.g., 500-2000 centigray) and hypo-fractionation (e.g., one to five fractions or treatment days). Radiotherapy is typically characterized by a low dose per fraction (e.g., 100-200 centigray) and hyper-fractionation (e.g., 30 to 45 fractions). For convenience, the terms "radiation treatment" and "radiation /therapy" are used interchangeably herein to mean radiosurgery and/or radiotherapy unless otherwise noted.

Associated with each radiation therapy system is an imaging system to provide in-treatment images that are used to set up and, in some examples, guide the radiation delivery procedure and track in-treatment target motion. MV imaging systems can place a detector opposite the therapeutic source to image the patient for setup and in-treatment images, while other approaches utilize distinct, independent image radiation source(s) and/or detector(s) for the patient set-up and in-treatment images. Target or target volume tracking during treatment is accomplished by comparing in-treatment images to prior or pre-treatment image information. Pre-treatment image information may comprise, for example, CT data, cone-beam CT (CBCT) data, magnetic resonance imaging (MRI) data, positron emission tomography (PET) data or 3D rotational angiography (3DRA) data, and any information obtained from these imaging modalities (for example and without limitation, digitally reconstructed radiographs (DRRs)).

There are typically two general goals in radiation therapy: (i) to deliver a highly conformal dose distribution to the target volume (by utilizing CT imaging to conform the delivery/dose of radiation to the shape of the target; normal tissue is spared as much as possible); and (ii) to deliver treatment beams with high accuracy throughout every treatment fraction. A third goal is to accomplish the two general goals in as little time per fraction as possible. Delivering a more conformal dose distribution may require, for example, the ability to deliver non-coplanar beams. Delivering treatment beams accurately may require the ability to track the location of the target volume intrafraction. The ability to increase delivery speed requires the ability to accurately, precisely, and quickly move the radiation source without hitting other objects in the room or the patient, and/or violating regulatory agency speed limitations.

CBCT has been proposed as an in-treatment imaging modality for use in conjunction with radiation treatment systems, in some cases as a kV imaging modality and in other cases as a MV (portal) imaging modality. CBCT imaging directly constructs a 3D volumetric image from 2D projections of the target volume and CBCT offers the ability to form a 3D image volume from a single gantry rotation (more specifically, a rotation of at least 180 degrees plus a fan beam angle) about the target volume. CBCT also provides for a more isotropic spatial resolution.

Dose calculation and treatment planning using CBCT requires that the CBCT images be of high quality and quantitative accuracy. However, when lateral, in-plane patient truncation occurs, artifacts and quantitative bias are introduced, making the CBCT image unsuitable for use in dose calculation and treatment planning. Although patient truncation can be reduced by patient positioning and scanning field-of-view (FOV) selection, automatic patient positioning and FOV selection may be challenging. For example, selection of a FOV that is smaller than the patient size and/or off-centered patient positioning within the FOV may result in lateral truncation. Additionally, selection of a FOV that is larger than sufficient requires a greater detector offset, a greater detector area unused to detect patient data, and less useful patient data for the same patient dose.

### Flat Panel Detection

Conventional CBCT systems use flat-panel detectors (FPDs) that principally detect x-rays through scintillation. Despite their commonality and general utility in CT applications, FPDs have some limitations. For example, FPD dynamic range (typically 16-bits) is typically insufficient to image some medically relevant features (e.g., lesions, especially those with low contrast). They also have poor quantum efficiency (typically only ~ 0.5 mm thick crystal) and slow readout (typically around 100 frames per second). Accordingly, there is a need for alternative systems and methods that use imaging technologies other than FPDs to improve, for example, image and energy resolution.

### BRIEF SUMMARY

Disclosed herein is a treatment apparatus. The treatment apparatus includes a rotatable gantry system positioned at least partially around a patient support and a first source of radiation coupled to the rotatable gantry system. The first source of radiation is configured to emit imaging x-ray radiation after pausing for a time interval between periodic emissions. The apparatus further includes a radiation detector configured to receive x-ray radiation from the first radiation source and generate tomographic data from the received radiation. The radiation detector is subject to a transient effect caused by at least one of polarization and charge trapping. The time interval is sufficiently long for the transient effect to substantially dissipate.

The treatment apparatus may further include a second source of radiation coupled to the rotatable gantry system. The second source of radiation may be configured for at least one of imaging radiation or therapeutic radiation, wherein the second source of radiation has an energy level more than the first source of radiation. The radiation detector associated with the first source may include a semiconductor material. The radiation detector may include an energy-resolved photon-counting detector. The radiation detector may be configured to discriminate energies of detected x-ray photons in the received radiation to generate the tomographic data.

The radiation detector may include a semiconductor material. A bias voltage may be provided to the semiconductor material when the radiation detector is receiving x-ray radiation. The bias voltage may be modified during the time interval between periodic emissions. Modification of the applied bias may include at least one of voltage reduction, nullification, and reversal. Modification of the bias voltage may be substantially applied to the interval between the periodic x-ray emission but can be initiated while the x-ray is still on and continue while the x-ray is restarted. Modification of the bias voltage may facilitate dissipation of the transient effect.

The semiconductor material may include at least one of silicon, cadmium, tellurium, and zinc. The radiation detector may include at least one of a cadmium zinc telluride (CdZnTe) detector, a silicon drift detector (SDD), a silicon PIN diode detector, and a cadmium telluride (CdTe) pixel detector.

The tomographic data may include images and may be collected at an imaging frame rate. The imaging frame rate may be 1000 frames per second or less. The imaging frame rate may be 700 frames per second or less. The first source of radiation may include a kilo-electron volt peak photon energy (keV) up to 150 keV and the second source of radiation may include a mega-electron volt peak photon energy (MeV) of 1 MeV or greater. The second source of radiation may include a peak energy of 6 MeV. The radiation detector may receive the radiation during a helical scan. The radiation detector may receive the radiation during an axial scan.

Also disclosed herein is a treatment apparatus including a first source of radiation configured to emit imaging x-ray radiation after pausing for a time interval between periodic emissions and a radiation detector configured to receive x-ray radiation from the first radiation source and generate tomographic data from the received radiation. The radiation detector is subject to a transient effect caused by at least one of polarization and charge trapping. The time interval is sufficiently long for the transient effect to substantially dissipate. The radiation detector includes a semiconductor material. A bias voltage is provided to the semiconductor material when the radiation detector is receiving x-ray radiation. The bias voltage is modified during the time interval between periodic emissions to facilitate dissipation of the transient effect.

The treatment apparatus may include a second source of radiation. The second source of radiation may be configured for at least one of imaging radiation or therapeutic radiation. The second source of radiation may have an energy level more than the first source of radiation.

The radiation detector may include an energy-resolved photon-counting detector. The radiation detector may be configured to analyze energies of detected x-ray photons in the received radiation to generate the tomographic data. The tomographic data may include images and may be collected at an imaging frame rate. The imaging frame rate may be 1000 frames per second or less. The imaging frame rate may be 700 frames per second or less.

Also disclosed herein is a treatment method. The method includes receiving, via a radiation detector, imaging x-ray radiation from a first radiation source, the first radiation source configured to emit the imaging x-ray radiation after pausing for a time interval between periodic emissions. The radiation detector is subject to a transient effect caused by at least one of polarization and charge trapping. The time interval is sufficiently long for the transient effect to substantially dissipate. The radiation detector includes a semiconductor material. The method further includes providing a bias voltage to the semiconductor material when the radiation detector is receiving x-ray radiation from the first radiation source. The method also includes modifying the bias voltage during the time interval between periodic emissions to facilitate dissipation of the transient effect.

The method may further include providing, via a second source of radiation, therapeutic radiation to a patient, wherein the second source of radiation has an energy level more than the first source of radiation. The first source of radiation may include a kilo-electron volt peak photon energy (keV) up to 150 keV and the second source of radiation may include a mega-electron volt peak photon energy (MeV) of 1 MeV or greater. The radiation detector may include an energy-resolved photon-counting detector.

The method may further include analyzing energies of detected x-ray photons in the received radiation to generate the tomographic data. The radiation detector may include at least one of a cadmium zinc telluride (CdZnTe) detector, a silicon drift detector (SDD), a silicon PIN diode detector, and a cadmium telluride (CdTe) pixel detector. The tomographic data may include images and may be collected at an imaging frame rate. The imaging frame rate may be 1000 frames per second or less. The imaging frame rate may be 700 frames per second or less.

Features that are described and/or illustrated with respect to one embodiment may be used in the same way or in a similar way in one or more other embodiments and/or in combination with or instead of the features of the other embodiments.

The descriptions of the invention do not limit the words used in the claims in any way or the scope of the claims or invention. The words used in the claims have all of their full ordinary meanings.

### BRIEF DESCRIPTION OF THE DRAWINGS

In the accompanying drawings, which are incorporated in and constitute a part of the specification, embodiments of the invention are illustrated, which, together with a general description of the invention given above, and the detailed description given below, serve to exemplify embodiments of this invention. It will be appreciated that illustrated element boundaries (e.g., boxes, groups of boxes, or other shapes) in the figures represent one embodiment of boundaries. In some embodiments, one element may be designed as multiple elements or that multiple elements may be designed as one element. In some embodiments, an element shown as an internal component of another element may be implemented as an external component and vice versa. Furthermore, elements may not be drawn to scale.
FIG. 1A shows an exemplary photon counting detector spectral CT (PCD-CT) detector setup 1 in the context of the present disclosure.
FIG. 1B shows an electron e⁻/hole h⁺ distribution under the influence of high x-ray 8 flux in setup 1.
FIG. 1C shows an accumulation of charge 9 that results from the configuration in FIG. 1B after exposure to high x-ray flux over time.
FIG. 1D shows polarization effects on the energy spectrum of detected photons in the form of "Normalized photon counts" at cathode 6 for different fluxes (mA).
FIG. 1E is a schematic of charge trapping by crystal defects in semiconductor PCDs.
FIG. 1F shows a comparison of the same detector exposed to continuous and pulsed mode x-ray.
FIG. 2A shows application of a pulse and intermittent bias method 200 in PCD-CT.
FIG. 2B is a field image of a detector element (single crystal of about 20mm x 20mm) operating with voltage bias pulsing.
FIG. 2C is a field image of the same detector element as 2B operating with continuous bias for the same flux and same amount of time as used in 2B.
FIG. 2D is a perspective view of an exemplary multimodal radiotherapy apparatus in accordance with one or more embodiments described herein.
FIG. 3 is a diagram of an exemplary multimodal radiotherapy device in accordance with one or more embodiments described herein.
FIG. 4 illustrates an exemplary radiation treatment environment in accordance with one or more embodiments described herein.
FIG. 5A is a flow chart depicting an exemplary method of using PCD-CT to obtain image data from a patient or object of interest.
FIG. 5B is a flow chart depicting another exemplary method of using PCD-CT to obtain image data from a patient or object of interest.
FIG. 6 is a flow chart depicting an exemplary method of combining scan data from multiple radiation modalities for online adaptive IGRT in accordance with one or more embodiments described herein.
FIG. 7 is a flow chart depicting an exemplary method of IGRT using a radiotherapy device in accordance with one or more embodiments described herein.
FIG. 8 is a block diagram depicting exemplary image-based pre-delivery steps/options in accordance with one or more embodiments described herein.
FIG. 9 is a block diagram depicting exemplary data sources that may be utilized during imaging and/or subsequent image-based pre-delivery steps in accordance with one or more embodiments described herein.
FIG. 10 is a flow chart depicting an exemplary method including patient setup or alignment using a radiotherapy device in accordance with one or more embodiments described herein.
FIG. 11 is a flow chart depicting an exemplary method including adaptive IGRT using a radiotherapy device in accordance with one or more embodiments described herein.

### DETAILED DESCRIPTION

The following includes definitions of exemplary terms that may be used throughout the disclosure. Both singular and plural forms of all terms fall within each meaning.

"Component," as used herein can be defined as a portion of hardware, a portion of software, or a combination thereof. A portion of hardware can include at least a processor and a portion of memory, wherein the memory includes an instruction to execute. A component may be associated with a device.

"Logic," synonymous with "circuit" as used herein, includes but is not limited to hardware, firmware, software and/or combinations of each to perform a function(s) or an action(s). For example, based on a desired application or needs, logic may include a software-controlled microprocessor, discrete logic such as an application specific integrated circuit (ASIC), or other programmed logic device and/or controller. Logic may also be fully embodied as software.

"Processor," as used herein includes, but is not limited to, one or more of virtually any number of processor systems or stand-alone processors, such as microprocessors, microcontrollers, central processing units (CPUs), and digital signal processors (DSPs), in any combination. The processor may be associated with various other circuits that support operation of the processor, such as random access memory (RAM), read-only memory (ROM), programmable read-only memory (PROM), erasable programmable read-only memory (EPROM), clocks, decoders, memory controllers, or interrupt controllers, etc. These support circuits may be internal or external to the processor or its associated electronic packaging. The support circuits are in operative communication with the processor. The support circuits are not necessarily shown separate from the processor in block diagrams or other drawings.

"Signal," as used herein includes, but is not limited to, one or more electrical signals, including analog or digital signals, one or more computer instructions, a bit or bit stream, or the like.

"Software", as used herein, includes but is not limited to one or more computer readable and/or executable instructions that cause a computer, processor, logic, and/or other electronic device to perform functions, actions, and/or behave in a desired manner. The instructions may be embodied in various forms such as routines, algorithms, modules, or programs including separate applications or code from dynamically linked sources or libraries.

While the above exemplary definitions have been provided, it is Applicant's intention that the broadest reasonable interpretation consistent with this specification be used for these and other terms.

As is discussed in more detail below, the disclosed technology relates to pulsed x-ray radiation in multimodal imaging/radiotherapy applications with associated devices and methods. The devices and methods employ pulsed radiation to improve energy resolution over image scan of continuous exposure such as modern CT systems. They include adjusting a period of the x-ray pulses and a bias applied to the detector, among other parameters, to diminish transient effects in the detector.

### Overview of Semiconductor-Based X-ray Detection

Semiconductor-based detection provides an alternative to FPDs and scintillation-based x-ray detection. Semiconductor detectors employ photon counting detector (PCD) technology that can use energy thresholding to collect energy information for individual x-ray photons. Other advantages are discussed in more detail below.

In particular, PCDs can be used in a promising therapeutic image modality called photon counting detector spectral (energy discriminating) CT (PCD-CT). PCD-CT can potentially produce tomographic images with enhanced and improved diagnostic information compared to that of energy integrating diagnostic CT. PCD-CT may achieve this, in part, by measuring each photon individually along with its associated energy. Dedicated electronics and/or software analyze this information to produce a more detailed image from x-ray photon detection.

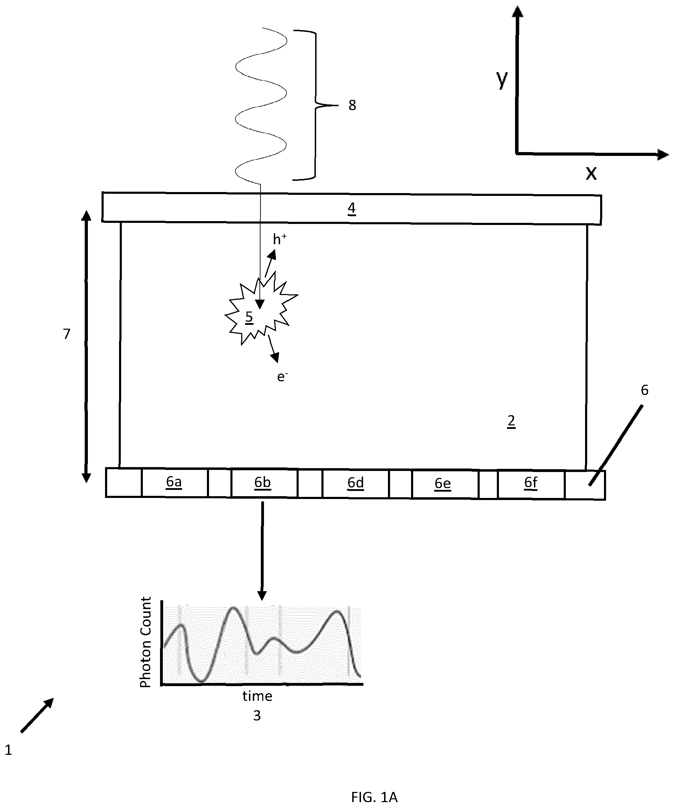

PCD-CT can use array(s) of radiation detector elements as pixels to create an image. FIG. 1A shows one such exemplary PCD-CT detector setup 1. Setup 1 represents one PCD detector that may be embedded in an array of similar detectors to create an image over a large patient area. Setup 1 includes semiconductor material 2, cathode 4, and an array of pixelated anodes 6a-6f together forming an anode block 6. Detection of incident x-rays 8 can generate signal 3 indicative of the number and energy of photons in the incoming radiation 8. Signals 3 may be spatially resolved at each anode 6a-6f, as well as each anode of the larger detector array. This can provide a spatially resolved x-ray image.

More specifically, X-ray detection via PCD setup 1 proceeds as follows. An individual x-ray photon 108 interacts 5 with semiconductor 2 thereby generating pairs of electrons e⁻ and holes h⁺ in semiconductor material 2. A bias voltage 7 applied between the cathode 4 and the collecting anodes 6a-6f creates an electric field within semiconducting material 2. The field generated by bias 7 causes the electrons e⁻ to drift toward anodes 6a-6f. The field also causes holes h⁺ to drift toward cathode 4. The time required for the electrons e⁻ to travel from the location of the x-ray interaction 5 to the anode 6, the "drift time," relates to the electric field strength along the path and to the structure/quality of the semiconductor material 2, as well as any electronic processes within material 2. An image can be formed from the signal 3 made by the electrons e⁻ collected at anodes 6a-6f. The signal 3 will be related to the energy of the original x-ray photon 8. As shown in FIG. 1A, signal 3 can include a cumulative representation of x-ray interactions with semiconductor material 2 over time. In principle, each of the anode pixels 6a-6f can generate its own signal 3. The data from these signals can be spatially resolved and compiled to create a tomographic image.

### Problems Using Semiconductor-Based Detection for Medical Imaging

Unfortunately, interpretation of detection events 5 represented in FIG. 1A can be complicated by processes occurring during x-ray detection. Such processes include polarization in the semiconductor material 2 and charge trapping effects occurring in its defects, for example. These problems and their mitigation are discussed in more detail below.

### Polarization

Polarization in the semiconductor material 2 during detection can negatively affect PCD-CT performance. Polarization can occur when high x-ray flux generates significant numbers of trapped or accumulated electrons e⁻ and/or holes h⁺ via interactions 5 sufficient to alter the field experienced by electrons e⁻ during drift. Since polarization is not well controlled, its effect on drift can make energy resolution of count data 3 difficult or impossible. These effects are cumulative and get worse over the course of an x-ray imaging session. They also worsen with increased x-ray flux. They can cause temporal drift of signal 3 and general signal instability.

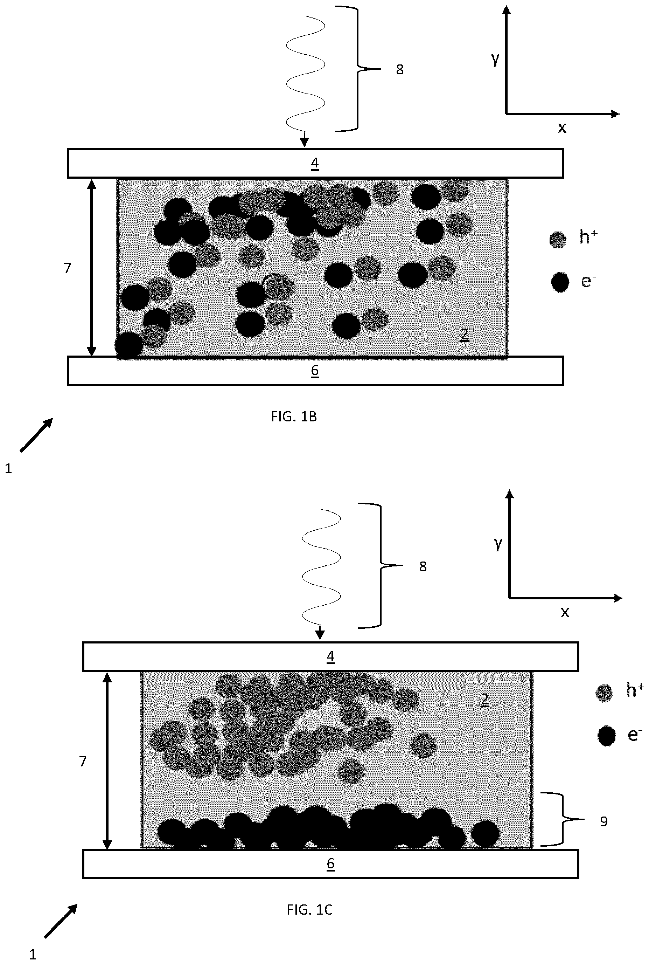

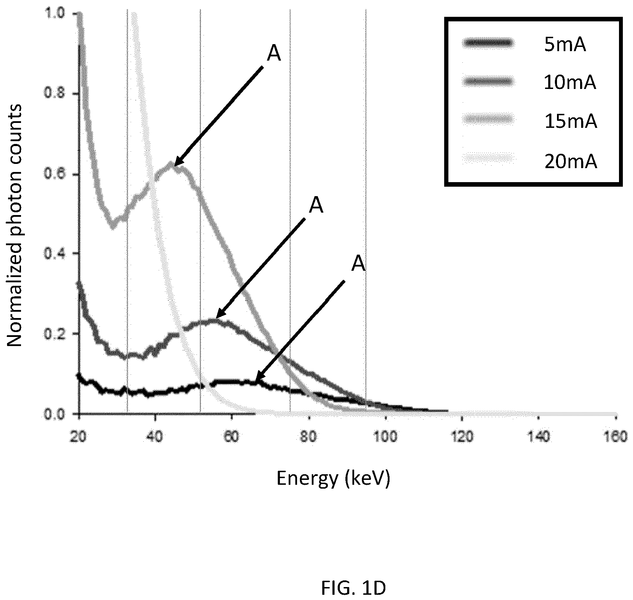

FIGs. 1B, 1C, and 1D illustrate polarization under high x-ray flux. FIG. 1B shows the electron e⁻/hole h⁺ distribution caused by the influence of high x-ray 8 flux. Both electrons e⁻ and holes h⁺ are created as the x-rays 8 interact with the semiconductor material 2 of the detector. As discussed above in the context of FIG. 1A, the electrons e⁻ will drift towards anode 6 and the holes h⁺ toward cathode 4 under the influence of applied voltage 7.

FIG. 1C shows an accumulation of charge 9 that results from this configuration after exposure to high x-ray flux for some time. As electrons e⁻ accumulate in the semiconductor material 2 close to the anode 6 (i.e., charge accumulation 9) and holes h⁺ close to the cathode 4, this polarized charge distribution primarily by the slow-moving holes, creates its own electric field. That field may counteract the field created by applied bias 7. More particularly, the field created by polarization can affect the drift of electrons e⁻ and holes h⁺ in material 2.

Under the conditions shown in FIGs.1C, drift times for electrons e⁻ are relatively poorly controlled. This makes quantitation of count signal 3 (FIG. 1A) difficult. It also renders its energetics ambiguous since E_{x-ray} is not well characterized. As discussed in more detail below, this can result in negative effects on signal 3. For example, it can reduce a detected "pulse height" representing an x-ray photon count. It can shift the energy of the pulse to energies lower than expected and/or report erroneous energy discrimination when multiple energy thresholds are used as in the case of a PCD. This can lead to both an underestimation of counted photons and a loss of accurate energy information relating to the detected photons.

Polarization can cause the analytics (i.e., software or hardware dedicated to collecting and interpreting signal 3) to lose or misinterpret individual x-ray photon counts. This data loss or misinterpretation can result in erroneous energy information for material decomposition. Incomplete charge collection can also result in erroneous energy registration and spectral distortion. In cases where the analytics assign detected data to an energy bin for energy resolving signal 3, events can be miscounted or mischaracterized when polarization erroneously increases or decreases energy with respect to binning thresholds. This may provide erroneous energy information for material decomposition purposes. Polarization can also lead more directly to artifacts in tomographic images.

FIG. 1D shows polarization effects on the energy spectrum of detected photons in the form of "Normalized photon counts" this can be a representative case of spectral shift using different fluxes (low to high) and over time when trapped charge is building up resulting to signal drift. FIG. 1D presents energy-resolved photon count data for objects with different x-ray fluxes in air. The key in FIG. 1D (identifying data for 5mA, 10mA, 15mA, and 20mA) differentiates signal levels. As the mA (flux) increases, the total flux curves (energy histograms) also increase. At the same time, the overall "mean" energy also shifts toward lower energy. For example, peak A of the "5mA" curve occurs at around 65 keV. The same peak A occurs around 40-45 keV on the "15 mA" curve. The downward energy shift for the "20 mA" curve is so severe that peak A can be identified. This shows how plots with higher x-ray flux (mA) experience spectral distortions.

### Charge Trapping

Another distortion in PCD-CT that can occur separately from, or in conjunction with, polarization is "charge trapping." This happens when crystal defects in the semiconductor material 2 of the detector (e.g., dislocations) ionize under the application of a constant bias 7 and immobilize charge carriers (e.g., electrons e⁻) and prevent them from reaching an electrode. As in the case of polarization discussed above, charge trapping can cause drift and collection in a detector system to deviate from expectations based on applied bias 7. Also charge trapping in certain locations (i.e., dislocations or other extended defects) can induce ionization of these locations and therefore create localized electric fields that can act as areas of polarization, as described previously. It can also cause erroneous energy registration and underestimation of photons counts resulting to spectral distortions (e.g., analogous to the spectral shift shown in FIG. 1D). This can lead to misinterpretation of image features and materials properties.

More specifically, charge trapping and ionization of crystal defects occurs after electrons e⁻ and holes h⁺ are injected by the detector 1 electrodes: cathode 4 and anode 6. This occurs both due to the application of a constant bias 7 and after electrons e⁻ liberated by x-ray events 5 when x-rays 8 enter the semiconductor material 2. Ionization of crystal defects by trapping of electrons e⁻ or holes h⁺ can occur under dark conditions (i.e., no x-rays 8) while the bias 7 is constantly applied to the detector 1. This happens, for example, during an idle state. After an x-ray event 8, x-ray generated electrons e⁻ drift through material 2 under the influence of bias 7. As this happens, the electrons e⁻ can become immobilized by these ionized, electrically active defects in the crystal structure of the semiconductor material 2. This slows and/or reduces electron e⁻ migration towards the collection anode 6 and limits the resulting signal 3 (FIG. 1A).

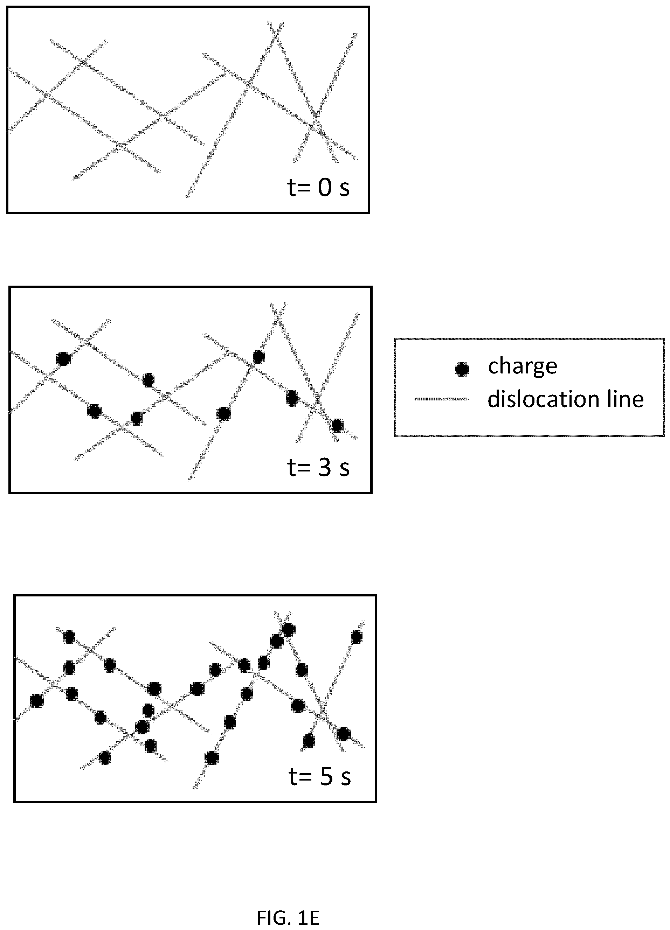

FIG. 1E shows the phenomenon schematically. FIG. 1E shows the progressive accumulation of charge in a network of electrically active crystal dislocations in the semiconductor portion 2 of a detector (e.g., a CdTe detector) under application of x-rays 8 and constant bias 7 (electric field), for three different durations t = 0, 3s, and 5s. As shown in the figure, the longer the x-ray exposure in conjunction with the application of constant bias, the more the charge trapping.

Crystal dislocations are a type of crystal defects that results from a localized deviation from a single crystalline structure. They can immobilize charge carriers, including electrons e⁻ and holes h⁺. Referring to FIG. 1E, at t = 0 s, x-ray flux (e.g., flux of x-rays 8 toward cathode 4 in FIG. 1A) has just been initiated. Because of the relatively short duration of flux at this point, no charge has yet accumulated in the dislocations. At t = 3 s, after substantial x-ray exposure, however, charge (electrons e⁻ or holes h⁺) has accumulated on the dislocation lines. At t = 5 s, additional x-ray exposure results in even more charge accumulating on the dislocation lines. As discussed above, this can create an electric field that interferes with the field created by applying voltage 7. Since the distribution of trapped charge may mirror or follow the pattern of dislocations in material 2, which is often random, the charge will be randomly distributed throughout the material. This can result in an uncertain and potentially unpredictable effect on the resultant electric field leading to drift, as explained in the context of FIG. 1A.

### Pulsed X-Ray

Pulsed x-ray is a method of image acquisition where the x-ray tube emits photons in pulsed sequence as small packets of energy information at short time increments. Pulsed x-ray is typically not applied in a CT setting because CT scans are typically too rapid for it to be implemented. Modern CTs operate at a very high rate of rotation (typically between 200 and 300 RPM with up to 2000 or more views per rotation). In contrast, radiation therapy devices (e.g., the Radixact system) tend to operate at much lower speeds, e.g., from few RPM to few tens of RPM. These lower speeds allow for the high energy linear accelerator generating therapeutic x-rays to be properly controlled so that it delivers precisely enough, and not more, radiation to the proper location.

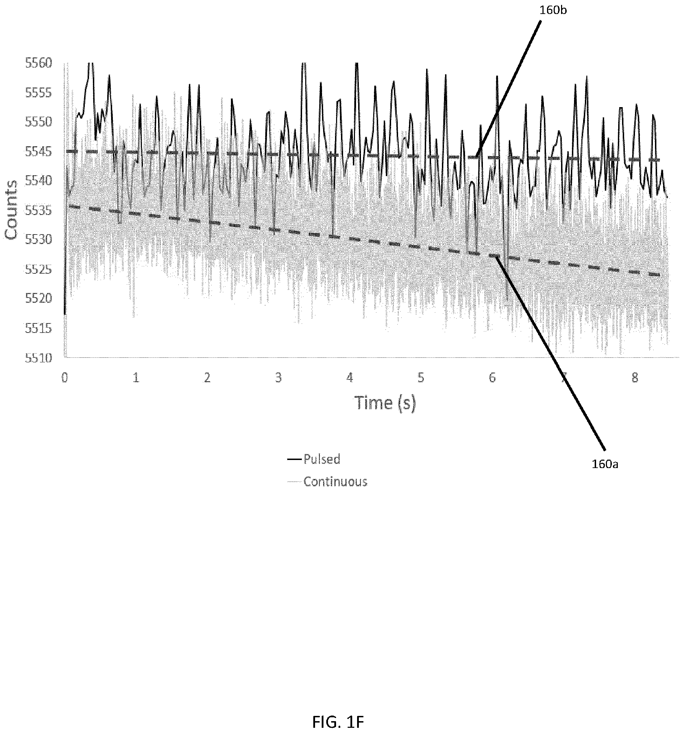

FIG. 1F shows the effect of pulsed and continuous x-ray radiation on detected intensity (count) data of the type that might be used to construct images in PCD-CT. In this example, x-rays are delivered to the sample at the rate of 30 pulses per second. Data shown in FIG. 1F is presented in terms of photon "counts" that can be energy resolved. The figure describes the average output counts from a selection of pixels of the detection system. Data in FIG. 1F is resolved over time to show the effect of polarization and/or charge trapping.

FIG. 1F clearly shows a net downward trend in photon counts with time for continuous radiation. This can be seen most readily via guiding line 160a that follows the average amplitude of the counts for the continuously applied x-ray. The continuous x-ray line 160a decreases from around 5535 counts at t = 0 to 5525 at t = 8 s. The result illustrates how polarization and charge trapping increase with the time of x-ray exposure, resulting in a reduction of the signal and decrease of measured counts. Data obtained from the pulsed x-ray source shows no substantial downward trend in photon counts with time. In that case, as shown by guiding line 160b, the counts remain relatively stable at around 5545 counts for the entire 8s measurement. This shows how pulsing an x-ray source has the potential to alleviate some of these effects, provided that the timing between pulses and the duration of pulses is well chosen.

### Overview of Solution and Advantages

Solutions disclosed herein make use of x-ray pulsing at predefined rates, as well as sequential bias pulsing (also known as bias refreshing). This ensures that intervals between pulses are long enough for defects like dislocations 162a-162c to de-ionize (release their trapped charge). When this happens, the locally distorted electric field within semiconductor material 2 can be restored and stabilized to approximate the original field created by an applied bias 7. It also delivers energy information in short increments per view angle, thus preventing pulse pileup. The solution also tailors x-ray pulsing to deliver packets of energy information over time intervals too short to allow onset of polarization.

### Pulse and Intermittent Bias Method Details

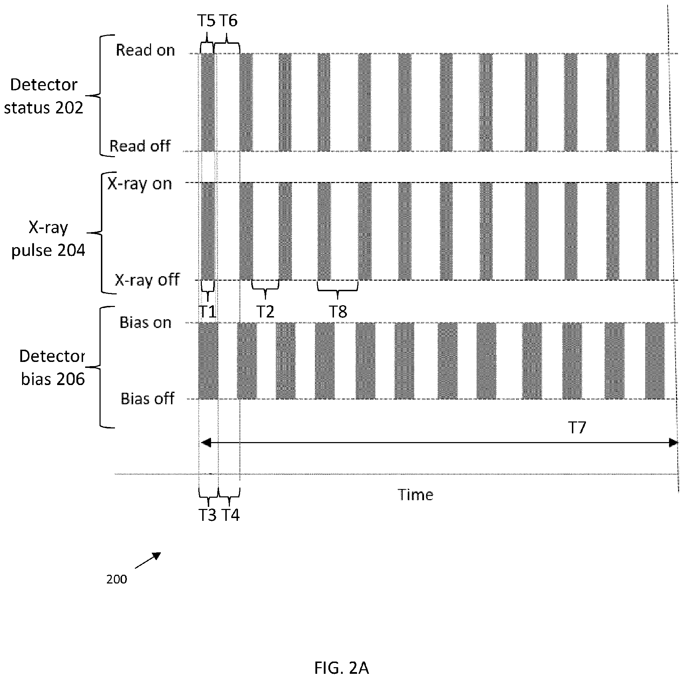

FIG. 2A shows application of a pulse and intermittent bias method 200 in PCD-CT. More specifically, the figure shows the detector status 202, generated x-ray pulse 204, and the bias imposed on the detector 206 resolved over time. For comparison with FIG. 1A, detector status 202 "read out on" corresponds to setting the detector 1 to acquire data from signal 3. Detector status 202 "read out off" corresponds to the detector 1 not acquiring data from signal 3. Generated x-ray pulsed signal 204 corresponds to x-ray 8. Detector bias 206 corresponds to bias 7. The x-axis of the plot in FIG. 2A is "Time." It is to be understood that the scale of this axis is variable and apparent dimensions on the plot are merely exemplary. For example, if the imaging frame rate of a PCD-CT system is 100 FPS, time would be scaled such that one x-ray pulse 204 duration shown in FIG. 2A is 10 ms. It can be appreciated that a large range in image frame rate can be used, depending on the specific application. The image frame rate can be chosen to simultaneously preserve information collected by the detector and allow sufficient time for the transient effects to substantially dissipate.

Additionally, FIG 2A shows instantaneous transition between the ON and OFF state for the various components. It will be appreciated that in practice, a certain amount of time is necessary to affect such transitions (e.g., turning off the x-ray generation or altering the voltage bias on a component). It is therefore a "time overlap" between those states can be tolerated while keeping substantially the same desired effects.

Table 1 provides a non-exhaustive list of variables that can be adjusted in method 200 to, among other things, improve the quality of detection and energy resolution in PCD-CT. Note that the variables in Table 1 are not necessarily independent.

| Label | Definition in method 200 |
|---|---|
| T1 | X-rays 8 are being delivered (pulse duration). |
| T2 | X-rays 8 are not being delivered (time between pulses). |
| T3 | Detector is subject to bias 7. |
| T4 | Detector is not subject to bias 7. |
| T5 | Detector is "read on." |
| T6 | Detector is "read off." |
| T7 | Duration of scan. |
| T8 | Period of x-ray signal (T1 + T2). |
| I | Intensity of x-ray pulses emitted from source. |
| E | Energy of x-ray pulses. |

| | |
|---|---|
| B | Value (V) of bias 7 on semiconductor material 2 in detector. |

As shown in FIG. 2A, each x-ray pulse 204 lasts for a time T1. In other words, T1 is the time when the x-ray source is on and providing x-rays incident to an object. In the exemplary configuration shown in FIG. 2A, the "read on" detector status 202 (T5) lasts for the same duration, i.e., T1. This ensures that the detector reads while x-rays are incident.

T2 is the time between x-ray pulses when the x-ray source is "dark," or not generating sending x-rays. T3 is the time duration over which bias 7 is applied to the detector. T4 is the time duration over which bias 7 is not applied to the detector (i.e., turned off such that it does not create an electric field within semiconducting material 2 and does not induce drift of charge carriers or additional trapping). T5 is the time when the detector is "read on," which means acquiring data for signal 3 (FIG. 1A). T6 is the time when the detector is "read off," which means not acquiring data. T7 is the duration of an exemplary (non-limiting) scan for the purposes of discussion and illustration. T8 is a period of the x-ray pulse 204 signal. Although it is sometimes convenient to refer to T8 as the period of the signal, T8 is not an independent variable. It depends on T1 and T2.

For example, T1 may be chosen to be short enough to prevent x-rays in one pulse from inducing substantial polarization in the semiconductor material 2 of the detector. The duration of T1 may be optimized to provide maximal amount of pulsed radiation without causing lasting charging or polarization. T2 may be chosen to be of sufficiently long duration such that any polarization and/or charge trapping that may occur has sufficient time to dissipate between successive x-ray pulses. The values of T2 may be chosen to be longer to compensate for a longer x-ray exposure (T1) and vice versa.

In another example, T3 and T4 may be chosen, as shown in FIG. 2A, to limit the application of bias 7 to semiconductor material 2 to times when an x-ray pulse is being delivered. As discussed above, it may be advantageous to turn off the bias 7 when x-rays 8 are not incident on the detector. This can allow both polarization and charge trapping to dissipate. As shown in the example in FIG. 2A, T3 may be greater than T1 applying bias for longer than the x-ray pulse. This can be done for a number of reasons, including to allow transients associated with the application of bias 7 to semiconducting material 2 to dissipate before applying x-ray radiation.

T5 and T6 may be chosen, as discussed above, to prevent detection of transients occurring during powering the x-ray source on or off. Alternatively, T5 and T6 may be chosen to maximize or minimize collected signal. T7 may be chosen to prevent charge accumulation over a scan. For example, a longer x-ray exposure time (T1) may be restricted to shorter scans (T4) with longer times between pulses (T2) so that charge does not accumulate in the detector. T7 is a duration of a hypothetical image scan encompassing multiple x-ray pulses.

As part of method 200, variables other than the timing variables T1-T8 may be adjusted and/or optimized for any of the reasons disclosed herein. For example, both the intensity I and the energy E of the incident x-rays 8 may be adjusted instead of or in tandem with T1-T8 to, for example, decrease charging and/or polarization effects. The voltage level of the bias B (element 7 in FIG. 1A) applied to the semiconductor material 2 in the detector may also be adjusted for similar reasons.

It is to be understood that combinations/configurations of T1-T8, I, E, and B, whether explicitly described herein or not, are within the scope of this disclosure. Moreover, the specific choice of T1-T8, I, E, and B depends on the desired result, the particular application, and the particular configuration of the x-ray detector system. The precise values of T1-T8, I, E, and B may vary according to the application and the specific structure (e.g., defect concentration), operation, and chemical identity of the detector.

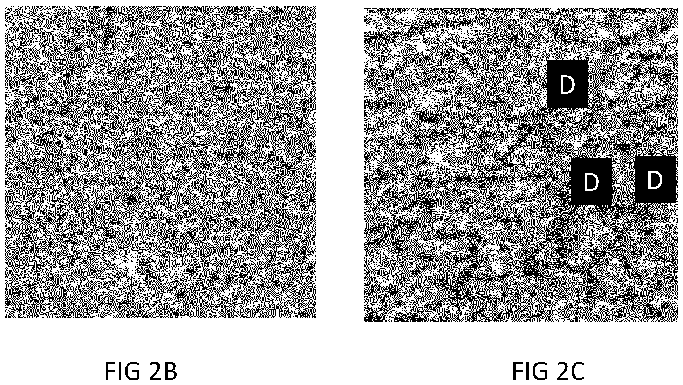

FIGs. 2B and 2C show application of these principles to improve detector performance. More specifically, FIGs. 2B show mitigating charge trapping using x-ray pulsing with appropriately chosen T1-T4. FIG. 2B shows the detector in pulsed x-ray application mode with the bias on. FIG 2C shows the same detector operating with the same flux and same amount of time at 2B with the bias off. FIG 2C shows dislocation lines D where charges are trapped. Trapped charge in lines D affects the collection of the information to create the image.

### Apparatus and Operation

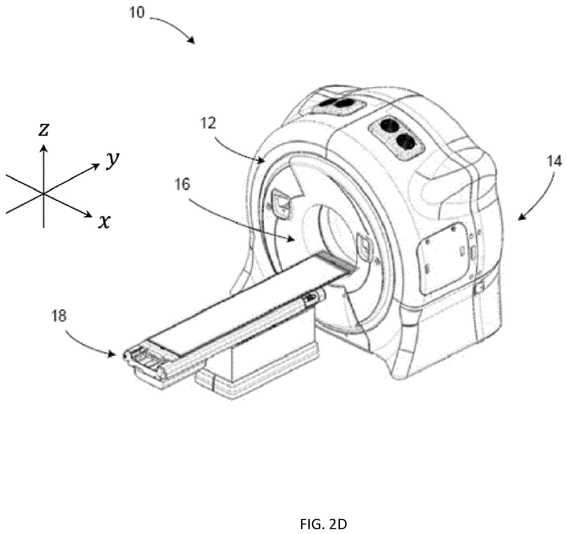

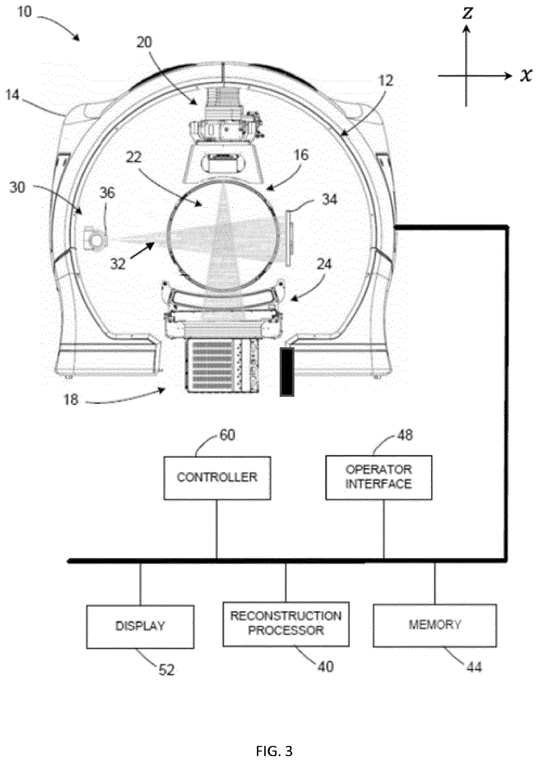

With reference to FIG. 2D and FIG. 3, a multimodal apparatus 10 is shown that can be used in PCD-CT. It will be appreciated that the multimodal apparatus 10 may be associated with and/or integrated into a radiotherapy device (as shown in FIG. 3) that can be used for a variety of applications. Multimodal apparatus 10 can be used, for example, in applications other than PCD-CT with the appropriate detector (e.g., an FPD).

Applications, include, but are not limited to IGRT, for example, as an IGRT delivery system. The multimodal apparatus 10 includes a rotatable gantry system, referred to as gantry 12, supported by or otherwise housed in a support unit or housing 14. Gantry herein refers to a gantry system that comprises one or more gantries (e.g., ring or C-arm) capable of supporting one or more radiation sources and/or associated detectors as they rotate around a target. For example, in one embodiment, a first radiation source and its associated detector may be mounted to a first gantry of the gantry system and a second radiation source and its associated detector may be mounted to a second gantry of the gantry system. In another embodiment, more than one radiation source and associated detector(s) may be mounted to the same gantry of the gantry system, including, for example, where the gantry system is comprised of only one gantry. Various combinations of gantries, radiation sources, and radiation detectors may be combined into a variety of gantry system configurations to image and/or treat the same volume within the same apparatus. For example, kV and MV radiation sources can be mounted on the same or different gantries of the gantry system and selectively used for imaging and/or treatment as part of an IGRT system. If mounted to different gantries, the radiation sources are able to rotate independently, but are still able to simultaneously image the same (or nearly the same) volume. A rotatable ring gantry 12 may be capable of 10 rpm or more.

The rotatable gantry 12 defines a gantry bore 16 into and through which a patient can be moved and positioned for imaging and/or treatment. In accordance with one embodiment, the rotatable gantry 12 is configured as a slip ring gantry to provide continuous rotation of radiation sources and associated radiation detector(s) while providing sufficient bandwidth for the high-quality imaging data received by the detector(s). A slip-ring gantry can eliminate gantry rotations in alternating directions in order to wind and unwind cables carrying the power and signals associated with the device. Such a configuration will allow for continuous helical computed tomography, including CBCT, even when integrated into an IGRT system.

A patient support 18 is positioned adjacent to the rotatable gantry 12 and configured to support a patient, typically in a horizontal position, for longitudinal movement into and within the rotatable gantry 12. The patient support 18 can move the patient, for example, in a direction perpendicular to the plane of rotation of the gantry 12 (along or parallel to the rotation axis of the gantry 12). The patient support 18 can be operatively coupled to a patient support controller for controlling movement of the patient and patient support 18. The patient support controller can be synchronized with the rotatable gantry 12 and sources of radiation mounted to the rotating gantry for rotation about a patient longitudinal axis in accordance with a commanded imaging and/or treatment plan. The patient support can also be moved in a limited range up and down, left and right once it is in the bore 16 to adjust the patient position for optimal treatment. Axes *x*, *y*, and z are shown, where, viewing from the front of the gantry 12, the x-axis is horizontal and points to the right, the *y*-axis points into the gantry plane, and the z-axis is vertical and points to the top. The *x-*, *y-*, and z-axes follow the right-hand rule.

As shown in FIG. 3, the multimodal apparatus 10 includes a low-energy radiation source (e.g., kV) 30 coupled to or otherwise supported by the rotatable gantry 12. In this embodiment, the low-energy radiation source 30 is a source of imaging radiation and emits a radiation beam (indicated generally as 32) for generating high-quality images. In this embodiment, the source of imaging radiation is an x-ray source 30, configured as a kilovoltage (kV) source (e.g., a clinical x-ray source having an energy level in the range of about 20 kV to about 150 kV). In one embodiment, the low energy radiation source comprises a kilo-electron volt peak photon energy (keV) up to 150 keV. As discussed extensively above, this low-energy radiation source 30 is configured to be able to deliver x-ray radiation in pulse mode. In particular, low-energy radiation source 30 is configured such that the parameters of its pulses (e.g., T1-T8, FIG. 2A) may be configurable. In principle, source 30 should allow any suitable parameter configuration. In certain variations, the parameters of source 30 may be adjustable "on the fly," remotely, and/or automatically.

The imaging radiation source can be any type of transmission source suitable for imaging. For example, the imaging radiation source may be, for example, an x-ray generating source (including for CT) or any other way to produce photons with sufficient energy and flux (such as, e.g., a gamma-source (e.g., Cobalt-57, energy peak at 122 keV), an x-ray fluorescence source (such as fluorescence source through Pb k lines, two peaks at about 70 keV and at about 82 keV), etc.). References herein to x-ray, x-ray imaging, x-ray imaging source, etc. are exemplary for particular embodiments. Other imaging transmission sources can be used interchangeably in various other embodiments. An x-ray detector 34 (e.g., a PCD) can be coupled to or otherwise supported by the rotatable gantry 12. The x-ray detector 34 is positioned to receive radiation from the x-ray source 30 and can rotate along with the x-ray source 30.

It will be appreciated that the x-ray detector 34 can take on a number of configurations without departing from the scope of the disclosed technology. For example, x-ray detector 34 can be configured a PCD, such as a silicon-based PCD. Suitable detectors also include energy-resolved photon-counting detector. Suitable detector types include silicon-based, cadmium zinc telluride (CdZnTe)-based, or cadmium telluride (CdTe)-based detectors. X-ray detector 34 may include a semiconductor material 2 that comprises any, or any combinations of, suitable semiconductor materials.

In some embodiments, x-ray detector 34 may include detectors other than PCDs. For example, detector 34 may include a flat-panel detector (e.g., a multi-row flat panel detector). Still other detector types and techniques known in the art may be included in detector 34 with departing from the scope of this disclosure.

Although FIGs. 2D and 3 depict a multimodal apparatus 10 with a radiation source 30 mounted to a ring gantry 12, other embodiments may include other types of rotatable imaging apparatuses, including, for example, C-arm gantries and robotic arm-based systems. In gantry-based systems, a gantry rotates the imaging radiation source 30 around an axis passing through the isocenter. Gantry-based systems include C-arm gantries, in which the imaging radiation source 30 is mounted, in a cantilever-like manner, over and rotates about the axis passing through the isocenter. Gantry-based systems further include ring gantries, for example, rotatable gantry 12, having generally toroidal shapes in which the patient's body extends through a bore of the ring/toroid, and the imaging radiation source 30 is mounted on the perimeter of the ring and rotates about the axis passing through the isocenter. In some embodiments, the gantry 12 rotates continuously. In other embodiments, the gantry 12 utilizes a cable-based system that rotates and reverses repeatedly.

A collimator or beamformer assembly (indicated generally as 36) is positioned relative to the x-ray source 30 to selectively control and adjust a shape of a radiation beam 32 emitted by the x-ray source 30 to selectively expose a portion or region of the active area of the x-ray detector 34. The beamformer can also control how the radiation beam 32 is positioned on the x-ray detector 34. In one embodiment, the beamformer 36 could have one degree/dimension of motion (e.g., to make a thinner or fatter slit). In another embodiment, the beamformer 36 can have two degrees/dimensions of motion (e.g., to make various sized rectangles). In other embodiments, the beamformer 36 may be capable of various other dynamically-controlled shapes, including, for example, parallelograms. All of these shapes may be dynamically adjusted during a scan. In some embodiments, blocking portions of the beamformer can be rotated and/or translated.

The beamformer 36 can be controlled to adjust the shape of the radiation beam 32 emitted by the x-ray source 30 dynamically in a number of geometries, including, but not limited to, a fan beam or cone beam having a beam thickness (width) as low as one detector row width or including multiple detector rows, which may be only a portion of the detector's active area. In various embodiments, the thickness of the beam may expose several centimeters of a larger detector active area. For example, from about 3 to about 4 centimeters (measured in the longitudinal direction in the detector plane) of a detector having an active area of from about 5 to about 6 centimeters may be selectively exposed to the imaging radiation 32. In this embodiment, from about 3 to about 4 centimeters of projection image data may be captured with each readout, with from about 1 to about 2 centimeters of unexposed detector area on one or each side, which may be used to capture scatter data, as discussed below.

In other embodiments, more or less of a portion of the active detector may be selectively exposed to the imaging radiation. For example, in some embodiments, the beam thickness may be reduced down to about two centimeters, one centimeter, less than one centimeter, or ranges of similar sizes, including with smaller detectors. In other embodiments, the beam thickness may be increased to about 4 centimeters, about 5 centimeters, greater than 5 centimeters, or ranges of similar sizes, including with larger detectors. In various embodiments, the ratio of exposed-to-active detector area may be from about 30% to about 90% or from about 50% to about 75%. In other embodiments, the ratio of exposed-to-active detector area may be from about 60% to about 70%. However, various other exposed and active area sizes or ratios of exposed-to-active detector area may be suitable in other embodiments. The beam and detector can be configured so that the shadowed region of the detector (active but not exposed to direct radiation) is sufficient to capture scatter data beyond the penumbra region.

Various embodiments may include an optimization of the features that control selective exposure of the detector (e.g., beam size, beam/aperture center, collimation, pitch, detector readout range, detector readout center, etc.) such that the measured data is sufficient for primary (exposed) and shadowed regions, but also optimized for speed and dosage control. The beamformer 36 shape/position and detector 34 readout range can be controlled such that the radiation beam 32 from the x-ray source 30 covers as much or as little of the x-ray detector 34 based on the particular imaging task and scatter estimation process being carried out, including, for example, combinations of narrow and wide axial field-of-view (aFOV) scans, as will be described in greater detail below. The apparatus 10 has the ability to acquire both single rotation cone beam and wide and narrow beam angle images, helical or other.

The beamformer 36 may be configured in a variety of ways that allow it to adjust the shape of the radiation beam 32 emitted by the x-ray source 30. For example, the beamformer 36 can be configured to include a set of jaws or other suitable members that define and selectively adjust the size of an aperture through which the radiation beam from the x-ray source 30 may pass in a collimated manner. In accordance with one exemplary configuration, the beamformer 36 can include an upper jaw and a lower jaw, where the upper and lower jaws are movable in different directions (e.g., parallel directions) to adjust the size of the aperture through which the radiation beam from the x-ray source 30 passes, and also to adjust the beam 32 position relative to the patient to illuminate only the portion of the patient to be imaged for optimized imaging and minimized patient dose.

In accordance with one embodiment, the shape of the radiation beam 32 from the x-ray source 30 can be changed during an image acquisition. Stated differently, in accordance with one exemplary implementation, the beamformer 36 leaf positions and/or aperture width can be adjusted before or during a scan. For example, in accordance with one embodiment, the beamformer 36 can be selectively controlled and dynamically adjusted during rotation of the x-ray source 30 such that the radiation beam 32 has a shape with sufficient primary/shadow regions and is adjusted to include only an object of interest during imaging (e.g., the prostate). The shape of the radiation beam 32 being emitted by the x-ray source 30 can be changed during or after a scan, depending on the desired image acquisition, which may be based on imaging and/or therapeutic feedback, as discussed in more detail below.

As shown in FIG. 3, the multimodal apparatus 10 may be integrated with a radiotherapy device that includes a high-energy radiation source (e.g., MV) 20 coupled to or otherwise supported by the rotatable gantry 12. In accordance with one embodiment, the high-energy radiation source 20 is configured as a source of therapeutic radiation, such as a high-energy source of radiation used for treatment of a tumor within a patient in a region of interest. In other embodiments, the high-energy radiation source 20 is also configured as a source of imaging radiation, or at least utilized as such. It will be appreciated that the source of therapeutic radiation can be a high-energy x-ray beam (e.g., MV x-ray beam), and/or a high-energy particle beam (e.g., a beam of electrons, a beam of protons, or a beam of heavier ions, such as carbon) or another suitable form of high-energy radiation. In one embodiment, the high-energy radiation source 20 comprises a mega-electron volt peak photon energy (MeV) of 1 MeV or greater. In one embodiment, the high-energy x-ray beam has an average energy greater than about 0.8 MeV. In another embodiment, the high-energy x-ray beam has an average energy greater than about 0.2 MeV. In another embodiment, the high-energy x-ray beam has an average energy greater than about 150keV. Generally, the high-energy radiation source 20 has a higher energy level (peak and/or average, etc.) than the low-energy radiation source 30.

In one embodiment, the high-energy radiation source 20 is a LINAC producing therapeutic radiation (e.g., MV) and the imaging system comprises an independent low-energy radiation source 30 producing relatively low intensity and lower energy imaging radiation (e.g., kV). In other embodiments, the therapeutic radiation source 20 could be a radioisotope, such as, for example, Co-60, which can generally have energy of greater than about 1 MeV. The high-energy radiation source 20 can emit one or more beams of radiation (indicated generally by 22) toward a region-of-interest (ROI) within a patient supported on the patient support 18 in accordance with a treatment plan.

In various embodiments, the high-energy radiation source 20 is utilized as a source of therapeutic radiation and a source of imaging radiation. As discussed in detail below, sources of radiation 20, 30 may be used in conjunction with one another to provide higher quality and better utilized images. References to the therapeutic radiation source 20 herein are to distinguish the high-energy radiation source 20 from the low-energy radiation source 30, which may be used only for imaging. However, references to the therapeutic radiation source 20 include embodiments where the therapeutic radiation source 20 (high-energy radiation source) can be utilized for therapy and/or imaging. In other embodiments, at least one additional radiation source can be coupled to the rotatable gantry 12 and operated to acquire projection data at a peak photon energy distinct from the peak photon energies of sources of radiation 20, 30.

Detector 24 can be coupled to or otherwise supported by the rotatable gantry 12 and positioned to receive radiation 22 from the therapeutic radiation source 20. The detector 24 can detect or otherwise measure the amount of radiation not attenuated and therefore infer what was in fact attenuated by the patient or associated patient ROI (by comparison to what was initially generated). The detector 24 can detect or otherwise collect attenuation data from different angles as the therapeutic radiation source 20 rotates around and emits radiation toward the patient.

It will be further appreciated that the therapeutic radiation source 20 can include or otherwise be associated with a beamformer or collimator. The beamformer associated with the therapeutic radiation source 20 can be configured in a number of ways, similar to the beamformer 36 associated with the imaging source 30. For example, a beamformer can be configured as a multi-leaf collimator (MLC), which can include a plurality of interlaced leaves operable to move to one or more positions between a minimally-open or closed position and a maximally-open position. It will be appreciated that the leaves can be moved into desired positions to achieve a desired shape of a radiation beam being emitted by the radiation source. In one embodiment, the MLC is capable of sub-millimeter targeting precision.

The therapeutic radiation source 20 may be mounted, configured, and/or moved into the same plane or a different plane (offset) than the imaging source 30. In some embodiments, scatter caused by simultaneous activation of the radiation sources 20, 30 may be reduced by offsetting the radiation planes.

In other embodiments, scatter can be avoided by interleaving the activations. For example, with simultaneous multimodal imaging, the acquisitions can be concurrent, without having concurrent individual pulses. In another embodiment, use of shadow-based scatter correction can be used, for example, to address the problem of MV scatter on a kV detector.

When integrated with a radiotherapy device, multimodal apparatus 10 can provide images that are used to set up (e.g., align and/or register), plan, and/or guide a radiation delivery procedure (treatment). Typical set-up is accomplished by comparing current (in-treatment) images to pre-treatment image information. Pre-treatment image information may comprise, for example, CT data, cone-beam CT data, MRI data, PET data or 3D rotational angiography (3DRA) data, and/or any information obtained from these or other imaging modalities. In some embodiments, the multimodal apparatus 10 can track in-treatment patient, target, or ROI motion.

A reconstruction processor 40 can be operatively coupled to detector 24 and/or detector 34. In one embodiment, the reconstruction processor 40 is configured to generate patient images based on radiation received by the detectors 24, 34 from the radiation sources 20, 30. It will be appreciated that the reconstruction processor 40 can be configured to be used to carry out the methods described more fully below. The apparatus 10 can also include a memory 44 suitable for storing information, including, but not limited to, processing and reconstruction algorithms and software, imaging parameters, image data from a prior or otherwise previously-acquired image (e.g., a planning image), treatment plans, and the like.

The multimodal apparatus 10 can include an operator/user interface 48, where an operator of the apparatus 10 can interact with or otherwise control the apparatus 10 to provide input relating to scan or imaging parameters and the like. The operator interface 48 can include any suitable input devices, such as a keyboard, mouse, voice-activated controller, or the like. The apparatus 10 can also include a display 52 or other human-readable element to provide output to the operator of the apparatus 10. For example, the display 52 can allow the operator to observe reconstructed patient images and other information, such as imaging or scan parameters, related to operation of the apparatus 10.

As shown in FIG. 3, the multimodal apparatus 10 includes a controller (indicated generally as 60) operatively coupled to one or more components of the apparatus 10. The controller 60 controls the overall functioning and operation of apparatus 10, including providing power and timing signals to the x-ray source 30 and/or the therapeutic radiation source 20 and a gantry motor controller that controls rotational speed and position of the rotatable gantry 12. It will be appreciated that the controller 60 can encompass one or more of the following: a patient support controller, a gantry controller, a controller coupled to the therapeutic radiation source 20 and/or the x-ray source 30, a beamformer controller, a controller coupled to the detector 24 and/or the x-ray detector 34, and the like. In one embodiment, the controller 60 is a system controller that can control other components, devices, and/or controllers.

In various embodiments, the reconstruction processor 40, the operator interface 48, the display 52, the controller 60 and/or other components may be combined into one or more components or devices.

The apparatus 10 may include various components, logic, and software. In one embodiment, the controller 60 comprises a processor, a memory, and software. By way of example and not limitation, a multimodal apparatus and/or radiotherapy system can include various other devices and components (e.g., gantries, radiation sources, collimators, detectors, controllers, power sources, patient supports, among others) that can implement one or more routines or steps related to imaging and/or IGRT for a specific application, wherein a routine can include imaging, image-based pre-delivery steps, and/or treatment delivery, including respective device settings, configurations, and/or positions (e.g., paths/trajectories), which may be stored in memory. Furthermore, the controller(s) can directly or indirectly control one or more devices and/or components in accordance with one or more routines or processes stored in memory. An example of direct control is the setting of various radiation source or collimator parameters (power, speed, position, timing, modulation, etc.) associated with imaging or treatment. An example of indirect control is the communication of position, path, speed, etc. to a patient support controller or other peripheral device. The hierarchy of the various controllers that may be associated with the apparatus can be arranged in any suitable manner to communicate the appropriate commands and/or information to the desired devices and components.

Moreover, those skilled in the art will appreciate that the systems and methods may be implemented with other computer system configurations. The illustrated aspects of the invention may be practiced in distributed computing environments where certain tasks are performed by local or remote processing devices that are linked through a communications network. For example, in one embodiment, the reconstruction processor 40 may be associated with a separate system. In a distributed computing environment, program modules may be located in both local and remote memory storage devices. For instance, a remote database, a local database, a cloud-computing platform, a cloud database, or a combination thereof can be utilized with apparatus 10.

Multimodal apparatus 10 can utilize an exemplary environment for implementing various aspects of the invention including a computer, wherein the computer includes the controller 60 (e.g., including a processor and a memory, which may be memory 44) and a system bus. The system bus can couple system components including, but not limited to the memory to the processor, and can communicate with other systems, controllers, components, devices, and processors. Memory can include read only memory (ROM), random access memory (RAM), hard drives, flash drives, and any other form of computer readable media. Memory can store various software and data, including routines and parameters, which may comprise, for example, a treatment plan.

The therapeutic radiation source 20 and/or x-ray source 30 can be operatively coupled to a controller 60 configured to control the relative operation of the therapeutic radiation source 20 and the x-ray source 30. For example, the x-ray source 30 can be controlled and operated simultaneously with the therapeutic radiation source 20. In addition, or alternatively, the x-ray source 30 can be controlled and operated sequentially with the therapeutic radiation source 20, depending on the particular treatment and/or imaging plan being implemented. For example, in various embodiments, the radiation sources 20, 30 can be operated such that the measured projection data from the radiation sources 20, 30 are acquired simultaneously (or essentially/nearly (quasi-) simultaneous, e.g., within about 50 ms of each other) or sequentially (e.g., separated by seconds, minutes, etc.).

It will be appreciated that radiation sources 20, 30 and detector(s) 24, 34 can be configured to provide rotation around the patient during an imaging and/or treatment scan in a number of ways. In one embodiment, synchronizing the motion and exposure of the source 20, 30 with the longitudinal motion of the patient support 18 can provide a continuous helical acquisition or scan of a patient image during a procedure. In addition to continuous rotation of the radiation sources 20, 30 and detector(s) 24, 34 (e.g., continuous and constant rotation of the gantry with constant patient motion speed), it will be appreciated that other variations can be employed without departing from the scope of the disclosed technology. For example, the rotatable gantry 12 and patient support can be controlled such that the gantry 12 rotates in a "back-and-forth" manner (e.g., alternating clockwise rotation and counterclockwise rotation) around a patient supported on the patient support (as opposed to continuously, as is described above) as the support is controlled to move (at a constant or variable speed) relative to the rotatable gantry 12. In another embodiment, with successive step-and-shoot circular scans, movement of the patient support 18 in the longitudinal direction (step) alternates with a scanning revolution by the rotatable gantry 12 (shoot) until the desired volume is captured. The multimodal apparatus 10 is capable of volume-based and planar-based imaging acquisitions. For example, in various embodiments, the multimodal apparatus 10 may be used to acquire volume images and/or planar images and execute the associated processing.

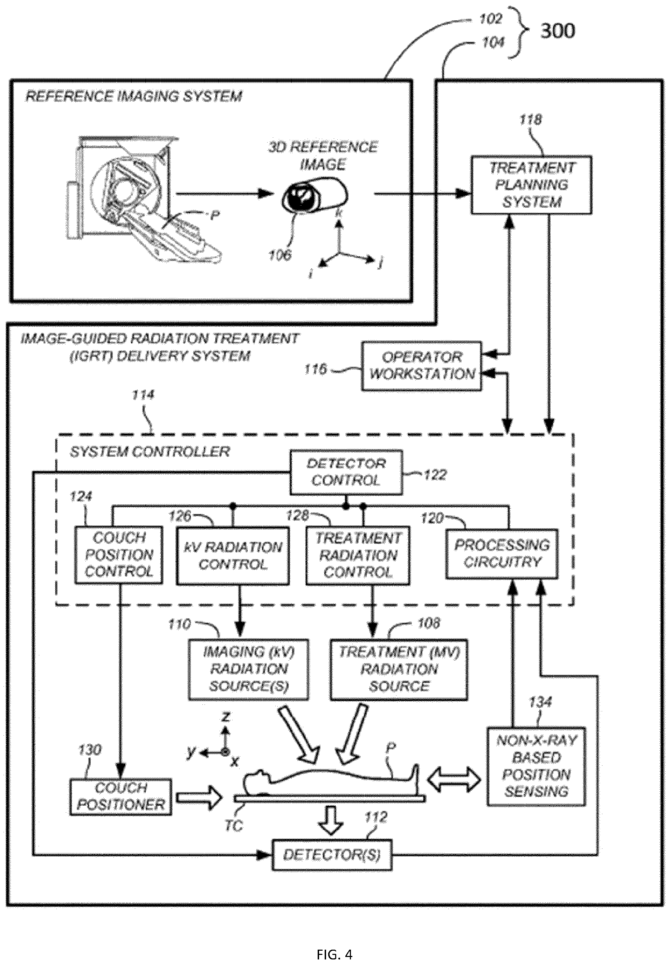

FIG. 4 illustrates an exemplary radiation treatment environment 300. The radiation treatment environment 300 includes a reference imaging system 102 and an IGRT system 104. The IGRT system 104 may comprise, for example, the multimodal apparatus 10 and its various components and devices as described above.

In one embodiment, the reference imaging system 102 can include a high precision volumetric imaging system such as, for example, a CT system or a MRI system. In view of cost and workflow considerations in many clinical environments, the reference imaging system 102 is often a general purpose tool used for a variety of different purposes in the clinic or hospital environment, and is not specifically dedicated to the IGRT system 104 or environment 300. Rather, the reference imaging system 102 may be located in its own separate room or vault and is purchased, installed, and/or maintained on a separate and more generalized basis than the IGRT system 104. Accordingly, for the embodiment of FIG. 4, the reference imaging system 102 is illustrated as being distinct from the IGRT system 104. In other embodiments, the reference imaging system 102 may be considered an integral component of the IGRT system 104. For example, the multimodal apparatus 10 has the capability to act as the reference imaging system 102 and the IGRT system 104.

In this embodiment, IGRT system 104 comprises a high-energy radiation treatment (MV) source 108 that selectively applies high-energy x-ray treatment radiation to a target volume of a patient P positioned on a patient support or treatment couch TC. The MV source 108 applies the treatment radiation under the control of system controller 114, and in one embodiment, more particularly a treatment radiation control subsystem 128. System controller 114 further comprises processing circuitry 120, a detector controller 122, a couch position controller 124, and a kV radiation controller 126, each programmed and configured to achieve one or more of the functionalities described further herein. One or more imaging (kV) radiation sources 110 selectively emit relatively low-energy x-ray imaging radiation under the control of kV radiation controller 126, the imaging radiation being captured by one or more detectors 112. One or more of the detectors 112 can capture high-energy x-ray treatment radiation from MV source 108 that has propagated through the target volume.

Each kV radiation source 110 and the MV radiation source 108 have a precisely measurable and/or precisely determinable geometry relative to the (*x*, *y*, *z*) coordinate system of the IGRT system 104 and/or treatment room since they are dynamically moveable.

A couch positioner 130 can be actuated by the couch position controller 124 to position the couch TC. In some embodiments, a non-x-ray based position sensing system 134 senses position and/or movement of external marker(s) strategically affixed to the patient, and/or senses position and/or movement of the patient skin surface itself, using one or more methods that do not involve ionizing radiation, such as optically based or ultrasonically based methods. IGRT system 104 further includes an operator workstation 116 and a treatment planning system 118.

In common clinical practice, treatment planning is performed on a pre-acquired treatment planning image or prior image data 106 generated by the reference imaging system 102. The pre-acquired treatment planning image 106 is often a high-resolution three-dimensional CT image acquired substantially in advance (e.g., one to two days in advance) of the one or more radiation treatment fractions that the patient will undergo. As indicated in FIG. 4 by the illustration of an (*i, j, k*) coordinate system for the pre-acquired treatment planning image 106, which is in contrast to the (*x, y, z*) treatment room coordinate system illustrated for the treatment room of the IGRT system 104, there is generally no pre-existing or intrinsic alignment or registration between the treatment planning image 106 coordinate system and the treatment room coordinate system. During the treatment planning process, a physician typically establishes a coordinate system (e.g., *i, j, k* in treatment planning image 106) within the treatment planning image, which may also be referred to herein as the planning image coordinate system or planning image reference frame. A radiation treatment plan is developed in the planning image coordinate system that dictates the various orientations, sizes, durations, etc., of the high-energy treatment radiation beams to be applied by the MV source 108 during each treatment fraction. Accurate delivery of therapeutic radiation to a target requires aligning the planning image coordinate system with the treatment room coordinate system, as the entire delivery and tracking system (if present) is calibrated to the treatment room coordinate system. It will be appreciated that this alignment does not need to be exact and further appreciated that couch adjustment or beam delivery adjustment can be used to account for offsets in the alignment between the two coordinate systems.

In embodiments, the treatment planning image 106 can be used, for example, to determine that the maximum FOV from a conventional CBCT configuration is sufficient, while in other embodiments, the treatment planning image 106 can be used to determine that an extended FOV as described herein is needed. Accordingly, the treatment planning image 106 can be used to determine the FOV and, accordingly, a detector offset and/or a lateral translation of the patient support to be used during the treatment, if any. The size of the FOV and amount of detector offset can, therefore, be optimized to prevent lateral truncation while improving detector usage and decreasing scanning time.

In one embodiment, immediately prior to each treatment fraction, under image guidance via the kV imaging radiation source(s) 110, including according to one or more of the embodiments described further herein below, image-based pre-delivery steps may be performed. For example, the patient can be physically positioned or aligned such that the planning image coordinate system (defined, for example and not by way of limitation, by a physician while creating a treatment plan on a CT image or planning image) is positioned into an initial alignment with the treatment room coordinate system, hereinafter termed an initial treatment alignment or initial treatment position. This alignment is commonly referred to as patient set up or patient alignment. Depending on the location of the target volume, the target volume can vary in position and orientation and/or can undergo volumetric deformations due to patient movement and/or physiological cycles such as respiration. As used herein, the term in-treatment alignment variation or in-treatment position variation is used to refer to the variations in position, orientation, and/or volumetric shape by which the current state of the target volume differs from the initial treatment alignment. By virtue of a known relationship between the treatment planning coordinate system and the treatment room coordinate system, the term in-treatment alignment variation can also be used to refer to the variations in position, orientation, or volumetric shape by which the current state of the target volume differs from that in the treatment planning coordinate system. More generally, the term initial treatment alignment or initial treatment position refers herein to the particular physical pose or disposition (including position, orientation and volumetric shape) of the body part of the patient upon patient setup at the outset of the treatment fraction.

A non x-ray based position sensing system 134 may also be provided. This non x-ray based position sensing system 134 may include, for example, external markers affixed in some manner to a patient's chest which move in response to respiration, which can precisely determine target location. Other mechanisms for monitoring respiration may also be used. Other non-respiratory position sensing systems 134 may also be used, including, for example, quasi static positioning, EKG for cardiac gating, etc. System 134 can correlate motion of the external markers with target motion, as determined from, for example, mono or stereoscopic x-ray projections. Non x-ray based position sensing system 134, therefore, can permit system controller 114 to monitor external marker motion, use the correlation model to precisely predict where the target will be located in real time (e.g., ^{~}60 Hz), and direct the treatment beam to the target. As treatment of the moving target progresses, additional x-ray images may be obtained and used to verify and update the correlation model.

As used herein, "registration" of medical images refers to the determination of a mathematical relationship between corresponding anatomical or other (e.g. fiducial) features appearing in those medical images. Registration can include, but is not limited to, the determination of one or more spatial transformations that, when applied to one or both of the medical images, would cause an overlay of the corresponding anatomical features. The spatial transformations can include rigid-body transformations and/or deformable transformations and can, if the medical images are from different coordinate systems or reference frames, account for differences in those coordinate systems or reference frames. For cases in which the medical images are not acquired using the same imaging system and are not acquired at the same time, the registration process can include, but is not limited to, the determination of a first transformation that accounts for differences between the imaging modalities, imaging geometries, and/or frames of reference of the different imaging systems, together with the determination of a second transformation that accounts for underlying anatomical differences in the body part that may have taken place (e.g., positioning differences, overall movement, relative movement between different structures within the body part, overall deformations, localized deformations within the body part, and so forth) between acquisition times.

Registration of images may be implemented between the reference imaging system 102 and the IGRT delivery system 104 and/or between the data and/or images derived from the various modalities of the multimodal IGRT delivery system 104, including the low energy source(s) 110 and the high energy source 108 (and their associated detectors 112). In particular, referring back to apparatus 10, registration may be implemented between data and/or images derived from radiation sources 20, 30 and detectors 24, 34.

The following flow charts and block diagrams illustrate exemplary configurations and methodologies associated with the multimodal radiation systems described above. The exemplary methodologies may be carried out in logic, software, hardware, or combinations thereof. In addition, although the procedures and methods are presented in an order, the blocks may be performed in different orders, including series and/or parallel. Further, additional steps or fewer steps may be used.

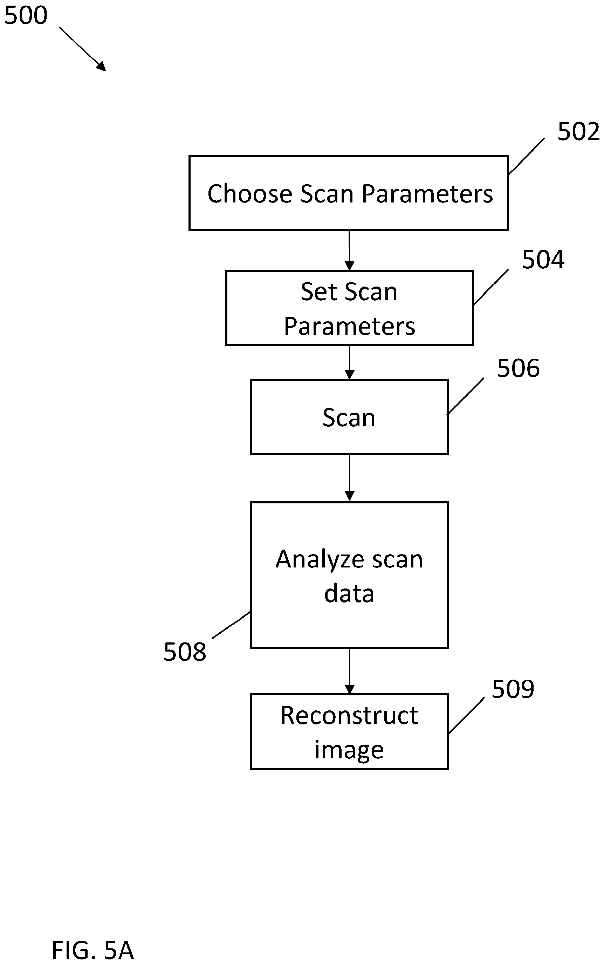

FIG. 5A is a flow chart depicting an exemplary method 500 of obtaining PCD-CT images using the principles of this disclosure. Although FIG. 5A and the other flow charts in this disclosure show sequences of steps, it is to be understood that this presentation is not limiting. When appropriate, steps shown in any of the flow charts disclosed herein, including in FIG. 5A, may be taken in a different order in accordance with this disclosure. Methods may also be practiced in accordance with this disclosure even if certain steps are omitted or added.

At step 502, parameters for imaging x-ray irradiation, including those for x-ray source 30 during scan implementation, are chosen. The parameters chosen in this step can include any of the parameters in Table 1 and FIG. 2A, in particular T1-T8, I, E, and B. Any suitable combination of parameters can be chosen for any of the reasons disclosed herein. For example, combinations of parameters T1-T8, I, E, and B can be chosen for the reasons discussed above in the context of method 200 and FIG. 2A.

In one particular variation, T1-T4 may be chosen such that pulse application of x-ray radiation 204 is of insufficient duration (T1) to cause substantial charging and/or polarization in the semiconductor material 2 of the PCD detector 34. Parameters T1-T8, I, E, and B may also be chosen so that the pause between applications of x-ray radiation (T2) is long enough for any trapped or accumulated charge to dissipate prior to the next pulsed application of x-ray radiation. As discussed above, parameters T1-T8, I, E, and B may be chosen in this step to optimize performance of the PCD 34 and the entire x-ray delivery setup. Variables that may be optimized include, but are not limited to, the amount and quality of data (x-ray photon counts), the resolution of energy in the detected energy spectrum, and the rate of data collection.

As discussed above, the values of the variables T1-T8, I, E, and B chosen will depend on the specific application, the x-ray setup (discussed further below), and the specific detector 34 used. Parameters specific to the detector 34 that can be important in deciding these values include the make/model of the detector, the structure of the detector (including crystal and dislocation structure), the chemical composition of the semiconductor material 2 in the detector 34, and the detector 34 operating specifications (e.g., speed of data acquisition, voltage tolerances, etc.).

In this step, prior image data (e.g., data from a reference image 106) may be provided to the controller 60. The prior image or other data may be used to help choose the parameters for the scan. For example, if a prior scan has insufficient resolution for a particular measurement, this may guide the selection of parameters T1-T8, I, E, and B to improve resolution. Prior image data may also be used to determine whether a position of the patient or object of interest should be adjusted or moved for the next scan.

At step 504, the scan parameters determined in step 502 are set. Typically, controller 60 sets the parameters for the entire apparatus 10, including T1-T8, I, E, and B. Therefore, this step may include applying a pulsed x-ray to the sample with intermittent biasing, as shown and discussed in the context of method 200. If it was determined in step 502 that the position of a patient or object of interest should be adjusted, that adjustment is made.

At step 506, the method 500 executes at least one scan. In embodiments, the scan can be a helical scan conducted with a scan configuration having the detector in a centered position and the patient support moving in a first longitudinal direction (e.g., the y-direction in FIG. 2D) relative to the gantry. Multiple scans may be taken at this step. For example, scans may be taken after altering the detector 34 position. Multiple scans may also be taken after altering the position of the patient or object of interest.

At step 508, the method 500 analyzes scan data. This step may include forming a combined data set using data from multiple scans taken in step 506. It may also include forming a combined data set using prior data. At step 509, the method 500 processes the combined data set, for example, to reconstruct an image.

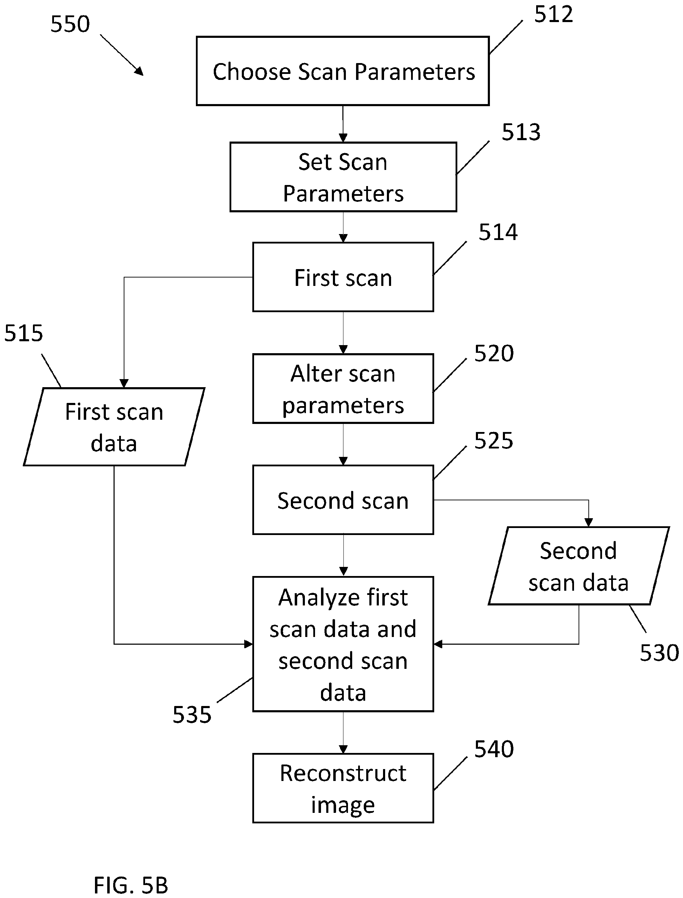

FIG. 5B is a flow chart depicting an exemplary method 550 that combines scan data from multiple radiation modalities, such as those described above for PCD-CT.

At step 512, parameters for imaging x-ray irradiation, including those for x-ray source 30 during scan implementation, are chosen. This step may include any of the features of step 502 of method 500 described above in the context of FIG. 5A. Scan parameters are then set in step 513, which may include any of the features of step 504 in method 500.

At step 514, the method 550 executes a first scan. In embodiments, the first scan is a helical scan conducted with a scan configuration having the detector in a first, centered position, and the patient support moving in a first longitudinal direction (e.g., the y-direction in FIG. 2D) relative to the gantry. The scan produces first scan data 515.

Next, at step 520, the method 550 analyzes the results from the first scan. The method 550 may, for example, determine whether parameters chosen in step 512 need to be altered. For example, if the image data appears subject to charging and/or polarization, parameters T1-T8, I, E, and B may be altered as described above in the context of method 200 to reduce these affects. This step may include altering any of the parameters in any of the ways disclosed herein to improve the image. In addition, a position of the detector 34 may also be changed. For example, the detector 34 can be laterally shifted with respect to the central beam from a first position to a second, offset position.

At step 525, the method 550 executes a second scan. The second scan can be identical to the first apart from any modifications made to the parameters in step 520. The scan produces second scan data 530.

At step 535, the method 550 analyzes the first scan data and the second scan data. In this step, the method 550 may select one of these sets of data as final data to create an image. Alternatively, the method 550 may use both data sets to form a combined final data set. Either way, the method produces final data for creating an image. At step 540, the method 500 processes the final data set, for example, to reconstruct an image.

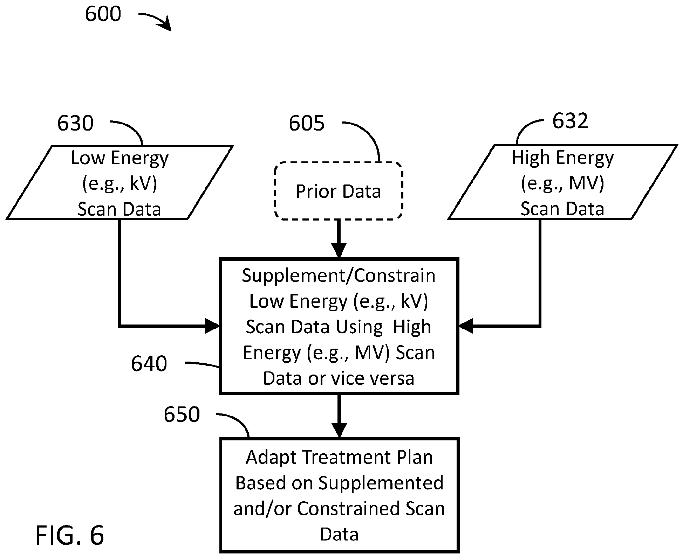

FIG. 6 is a flow chart depicting an exemplary method 600 of combining scan data from multiple radiation modalities for online adaptive IGRT. Prior data 605 is from known prior images, treatment plans, objects, models, etc. In some embodiments, a scan configuration (including scan designs for each modality of the multimodal system) may be determined and scans executed according to method steps 615 and 630 of method 600 to generate the scan data 630, 632. The low energy scan data 630, for example, may be from a low energy kV scan. The high energy scan data 632, for example, may be from a high-energy MV scan. At step 640, the method 600 makes use of or combines the low energy scan data 630 and the high energy scan data 632 to supplement and/or constrain the other scan data 630, 632. Then at step 650, the method 600 adapts the treatment plan based on the supplemented and/or constrained other scan data.

In some embodiments, fluence field modulation imaging includes supplementing missing MV view information with orthogonal kV projections. In some embodiments involving online 3D kV imaging, MV treatment projections are used as constraints on a quantitative image of electron density. In one embodiment, the online quantitative image is used for online dose calculation. In another embodiment, the calculated dose is used in an offline adaptive workflow. In another embodiment, the calculated dose is used in a quality assurance workflow.

In some embodiments, the above methods can be executed simultaneously or in an interleaved manner based on a preferred workflow. For example, a multimodal scan can be performed and the resulting scan data utilized for two or more of the various features and benefits described above.

When the above apparatus and methods are used in the projection domain, it can be applied on each projection view, where each projection view is a planar image. Various embodiments can utilize different scan geometries, detector positioning (including offset detectors), and/or beamformer window shapes.

As is discussed above, aspects of the disclosed technology can be utilized in a radiotherapy device and methods that make use of multimodal radiation sources, including integrated low energy (e.g., kV) and high energy (e.g., MV) sources for use in conjunction with or as part of IGRT. In accordance with one embodiment, the image acquisition methodology can include or otherwise makes use of a helical source trajectory (e.g., a continuous source rotation about a central axis together with longitudinal movement of a patient support through a gantry bore) or a circular scan, together with fast slip ring rotation, for example, to provide kV CT imaging on a radiation therapy delivery platform.

In some embodiments, it will be appreciated that any potential increased scan time associated with multiple beam rotations to complete a volume image can be mitigated or otherwise offset by high kV frame rates, high gantry rates, and/or sparse data reconstruction techniques. It will be further appreciated that the above-described provision of a selectively controllable collimator/beamformer allows for a system where a user can trade off or otherwise vary image acquisition time versus image quality, depending on the specific application and/or clinical need. It also will be appreciated that the radiotherapy delivery device can be controlled to provide half- or single-rotation cone beam CT scans (with potential reduced image quality) with fast image acquisition time (e.g., for motion tracking), as well as circular or continuous helical acquisition with a narrow/slit fan beam with longer acquisition time, but increased image quality. One or more optimization processes are also applicable to all of the above embodiments to determine scan designs, determine beam positioning, determine readout range, estimate scatter, etc.

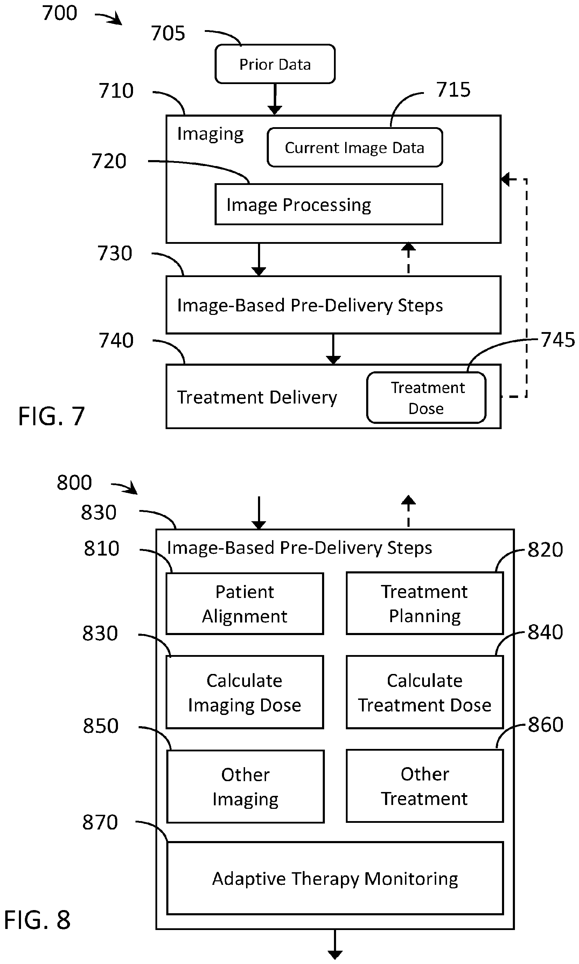

FIG. 7 is a flow chart depicting an exemplary method 700 of IGRT using a radiotherapy device (including, e.g., multimodal apparatus 10). Prior data 705 can include images of the patient (e.g., a prior image, which may be a previously-acquired planning image, including a prior CT image, as discussed above), treatment plans, phantom information, models, a priori information, etc. In some embodiments, the prior data 705 is generated by the same radiotherapy device, but at an earlier time. At step 710, imaging of a patient is performed using a source of radiation (e.g., kV radiation from source 30 and/or MV radiation from source 20 of multimodal apparatus 10). In various embodiments, imaging comprises a helical or circular scan with a fan or cone beam geometry. Step 710 can produce high-quality (HQ) image(s) or imaging/scan data 715 using the techniques described above. In some embodiments, image quality may be adjusted to optimize a balance between image quality/resolution and dosage. In other words, not all images need to be of the highest quality or image quality may be adjusted to optimize or trade off a balance between image quality/resolution and image acquisition time. Imaging step 710 also includes image processing 720 to generate patient images based on the imaging/scan data 715 (e.g., in accordance with methods described above). Although image processing step 720 is shown as part of imaging step 710, in some embodiments image processing step 720 is a separate step, including where image processing is executed by separate devices.

Next, at step 730, one or more image-based pre-delivery steps, discussed below, are performed based at least in part on the imaging/scan data 715 from step 710. As discussed in more detail below, step 730 can include determining various parameters associated with the therapeutic treatment and (subsequent) imaging planning. In some embodiments, image-based pre-delivery steps (730) may require more imaging (710) before treatment delivery (740). Step 730 can include adapting a treatment plan based on the imaging data 715 as part of an adaptive radiotherapy routine. In some embodiments, image-based pre-delivery steps 730 may include real-time treatment planning. Embodiments may also include simultaneous, overlapping, and/or alternating activation of the imaging and therapeutic radiation sources, as described above. Real-time treatment planning may involve any or all of these types of imaging and therapeutic radiation activation techniques (simultaneous, overlapping, and/or alternating).

Next, at step 740, therapeutic treatment delivery is performed using a source of high-energy radiation (e.g., MV radiation from therapeutic radiation source 20). Step 740 delivers a treatment dose 745 to the patient according to the treatment plan. In some embodiments, the IGRT method 700 may include returning to step 710 for additional imaging at various intervals (e.g., between fractions), followed by image-based pre-delivery steps (730) and/or treatment delivery (740) as required. In this manner the high-quality imaging data 715 may be produced and utilized during IGRT using one apparatus 10 that is capable of adaptive therapy. As mentioned above, steps 710, 730, and/or 740 may be executed simultaneously, overlapping, and/or alternating.

Delivering treatment beams accurately requires the ability to identify and/or track the location of the target volume intrafraction with high-quality images. The ability to increase delivery speed requires the ability to accurately, precisely, and quickly move the radiation source according to the treatment plan.

FIG. 8 is a block diagram 800 depicting exemplary image-based pre-delivery steps/options that may be associated with step 730 above. It will be appreciated that the above-described multimodal apparatus 10 (e.g., as part of a radiotherapy device) can generate low energy and high energy images that can be used in a variety of ways, including for image-based pre-delivery steps (730), without departing from the scope of the present invention. For example, images 715 generated by the radiotherapy device can be used to setup or align a patient prior to treatment (810). Patient alignment can include correlating or registering the current imaging data 715 with imaging data associated with earlier pre-treatment scans and/or plans, including the treatment plan. Patient alignment can also include feedback on the physical position of the patient relative to the radiation source to verify whether the patient is physically within the range of the delivery system. If necessary, the patient can be adjusted accordingly. In some embodiments, patient alignment imaging may purposely be of lesser quality to minimize dosage but provide adequate alignment information. An exemplary patient alignment process is described below.

Images generated by the multimodal apparatus 10 can also be used for treatment planning or re-planning (820). In various embodiments, step 820 can include confirming the treatment plan, modifying the treatment plan, generating a new treatment plan, and/or choosing a treatment plan from a set of treatment plans (sometimes referred to as "plan of the day"). For example, if the imaging data 715 shows that the target volume or ROI is the same as when the treatment plan was developed, then the treatment plan can be confirmed. However, if the target volume or ROI is not the same, re-planning of the therapeutic treatment may be necessary. In the case of re-planning, because of the high quality of the imaging data 715 (generated by the multimodal apparatus 10 at step 710), the imaging data 715 may be used for treatment planning or re-planning (e.g., generating a new or modified treatment plan). In this manner, pre-treatment CT imaging via a different device is not necessary. In some embodiments, confirming and/or re-planning may be an ongoing procedure before and/or after various treatments.

In accordance with another exemplary use case, images generated by the multimodal apparatus 10 can be used to calculate imaging dose (830), which may be used for ongoing determinations of total dose to the patient and/or for subsequent imaging planning. The quality of subsequent imaging may also be determined as part of the treatment planning, for example, to balance quality and dosage. In accordance with another exemplary use case, images generated by the multimodal apparatus 10 can be used to calculate treatment dose (840), which may be used for ongoing determinations of total dose to the patient and/or may be included as part of treatment planning or re-planning.

In accordance with other exemplary use cases, images generated by the multimodal apparatus 10 can be used in connection with planning or adjusting other imaging (850) and/or other treatment (860) parameters or plans, including, for example, as part of adaptive therapy and/or treatment plan generation. In accordance with another exemplary use case, images generated by the multimodal apparatus 10 can be used in connection with adaptive therapy monitoring (870), which can include monitoring treatment delivery and adapting as required, including re-planning.

It should be appreciated that the image-based pre-delivery steps (730) are not mutually exclusive. For example, in various embodiments, calculate treatment dose (840) can be a step by itself and/or can be part of adaptive therapy monitoring (870) and/or treatment planning (820). In various embodiments, the image-based pre-delivery steps (730) can be performed automatically and/or manually with human involvement.

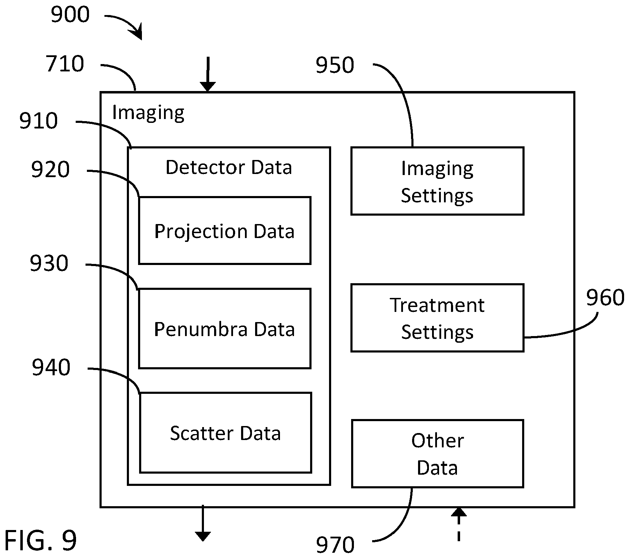

FIG. 9 is a block diagram 900 depicting exemplary data sources that may be utilized during imaging (710) and/or subsequent image-based pre-delivery steps (730). Detector data 910 represents the data received by the radiation detectors 24, 34. The projection data 920 is the data generated by the radiation incident in the collimated beam area, referred to above as the active region. The penumbra data 930 is the data generated by the radiation incident in the penumbra area. The scatter data 940 is the data generated by the radiation incident in the peripheral area outside of the penumbra area and/or the determined scatter as described in greater detail in U.S. Patent Application Publication No. 2020/0170598, now U.S. Patent No. 11,154,269 issued October 6, 2021, entitled "Multimodal Radiation Apparatus and Methods," filed on November 25, 2019, the entire contents of which is hereby incorporated by reference herein. In another embodiment, the scatter data 940 can be used to determine the residual effect of the scatter from the therapeutic radiation source 20 (e.g., MV) when the two sources 20, 30 are operated simultaneously or in an interleaved manner.

In this manner, the penumbra data 930 and/or the scatter data 940 may be utilized to improve the quality of the images generated by the imaging step 710. In some embodiments, the penumbra data 930 and/or the scatter data 940 may be combined with the projection data 920 and/or analyzed in view of the applicable imaging settings 950, treatment settings 960 (e.g., if simultaneous imaging and/or treatment radiation), and any other data 970 associated with the multimodal apparatus 10 at the time of the data collection at the detectors 24, 34. In other embodiments, the data may be used for the treatment planning step 930.

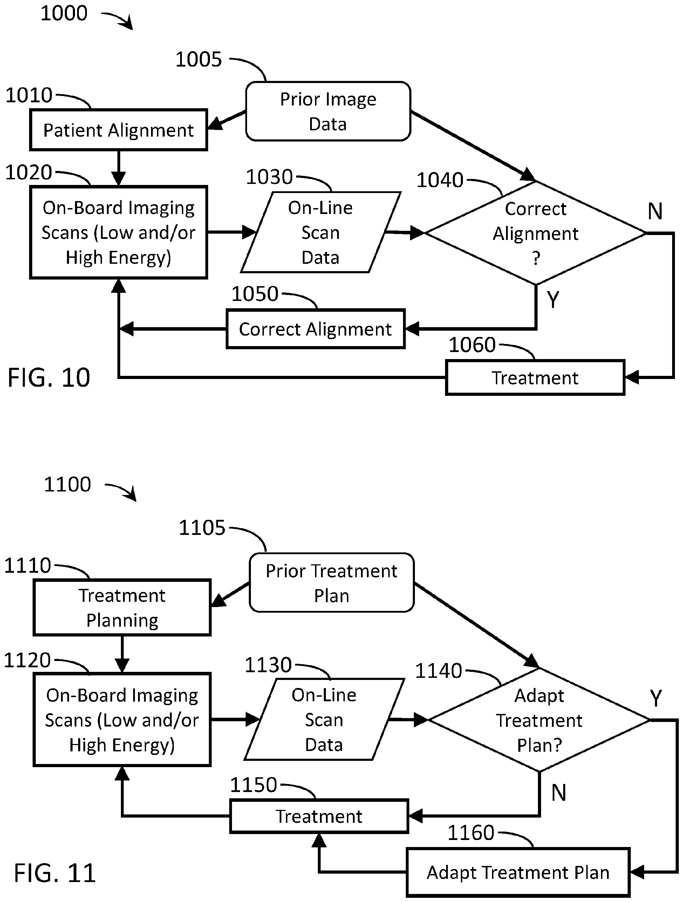

FIGS. 10 and 11 depict exemplary embodiments of imaging 710, image-based pre-delivery steps 730, and treatment delivery 740 use cases, including examples of various forward and feedback sequences associated with on-board imaging using multimodal apparatus 10 during IGRT, including use of the methods described above.

FIG. 10 is a flow chart depicting an exemplary method 1000 including patient setup or alignment using a radiotherapy device (including, e.g., multimodal apparatus 10). Prior data 1005 can include images of the patient (e.g., a prior image, which may be a previously-acquired planning image, including a prior CT image, as discussed above). In some embodiments, the prior data 1005 is generated by the same radiotherapy device, but at an earlier time. At step 1010, an initial or preliminary alignment of the patient can be performed. Then, at step 1020, on-board imaging scans are performed using a low and/or high energy radiation source (e.g., kV radiation from source 30 and/or MV radiation from source 20) of multimodal apparatus 10, including, for example, as described in step 710 above. In various embodiments, on-board imaging comprises a helical or circular scan with a fan or cone beam geometry. Step 1020 produces on-line imaging/scan data 1030 using the techniques described above. Imaging step 1020 can also include image processing to generate patient images based on the imaging/scan data 1030 (e.g., in accordance with methods described above), including, for example, as described in block 720 above.

Next, step 1040 determines if an alignment correction is needed based at least in part on the on-line scan data 1030, including, for example, as described in block 810 above. If an alignment correction or adjustment is needed, the method 1000 proceeds to step 1050 for an alignment correction, based at least in part on the on-line scan data 1030. Then, after the alignment correction, the method can return to step 1020 for additional imaging as a confirmation or further refinement loop. If an alignment correction or adjustment is not needed from step 1040, the method 1000 proceeds to step 1060 for treatment delivery. Then, after treatment, the method 1000 can return to step 1020 for additional imaging as a confirmation or further refinement loop.

FIG. 11 is a flow chart depicting an exemplary method 1100 including adaptive IGRT using a radiotherapy device (including, e.g., multimodal apparatus 10). Prior treatment plans 1105 can include treatment plans as well as images of the patient (e.g., a prior image, which may be a previously-acquired planning image, including a prior CT image, as discussed above), phantom information, models, a priori information, etc. In some embodiments, the prior data 1105 is generated by the same radiotherapy device, but at an earlier time. At step 1110, an initial or preliminary treatment plan can be adopted, for example, based on the prior treatment plan and any additional information. Then, at step 1120, on-board imaging scans are performed using a low and/or high energy radiation source (e.g., kV radiation from source 30 and/or MV radiation from source 20) of multimodal apparatus 10, including, for example, as described in step 710 above. In various embodiments, on-board imaging comprises a helical or circular scan with a fan or cone beam geometry. Step 1120 produces on-line imaging/scan data 1130 using the techniques described above. Imaging step 1120 can also include image processing to generate patient images based on the imaging/scan data 1130 (e.g., in accordance with methods described above), including, for example, as described in block 720 above.

Next, step 1140 determines if the treatment plan needs to be re-planned or adapted based at least in part on the on-line scan data 1130, including, for example, as described in block 820 above. If a treatment plan does not need to be adapted or re-planned, the method 1100 proceeds to step 1150 for treatment delivery. If a treatment plan does need to be adapted or re-planned, the method 1100 proceeds to step 1160 for adapting the treatment plan, based at least in part on the on-line scan data 1130. Then, after adapting the treatment plan, the method can proceed to step 1150 for treatment delivery. Then, after treatment, the method 1100 can return to step 1120 for additional imaging as a confirmation or further refinement loop.

In some embodiments, methods 1000, 1100 and other methods can be executed simultaneously or in an interleaved manner based on a preferred workflow. For example, an on-board imaging scan can be performed and utilized as both the 1020 and 1120 scans to confirm treatment and continued alignment at the same time using the same data. In other embodiments, two or more of the image-based pre-delivery steps 730 can be executed simultaneously or in an interleaved manner based on a preferred workflow, including where the same imaging data 715 and/or image processing 720 is utilized for more than one of the image-based pre-delivery steps 730.

Although the disclosed technology has been shown and described with respect to a certain aspect, embodiment or embodiments, it is obvious that equivalent alterations and modifications will occur to others skilled in the art upon the reading and understanding of this specification and the annexed drawings. In particular regard to the various functions performed by the above described elements (components, assemblies, devices, members, compositions, etc.), the terms (including a reference to a "means") used to describe such elements are intended to correspond, unless otherwise indicated, to any element which performs the specified function of the described element (i.e., that is functionally equivalent), even though not structurally equivalent to the disclosed structure which performs the function in the herein illustrated exemplary aspect, embodiment or embodiments of the disclosed technology. In addition, while a particular feature of the disclosed technology may have been described above with respect to only one or more of several illustrated aspects or embodiments, such feature may be combined with one or more other features of the other embodiments, as may be desired and advantageous for any given or particular application.

The application, furthermore, provides the following embodiments.
1. A treatment apparatus, comprising:
   a rotatable gantry system positioned at least partially around a patient support;
   a first source of radiation coupled to the rotatable gantry system, the first source of radiation configured to emit imaging x-ray radiation after pausing for a time interval between periodic emissions; and
   a radiation detector configured to receive x-ray radiation from the first radiation source and generate tomographic data from the received radiation, wherein:
      the radiation detector is subject to a transient effect caused by at least one of polarization and charge trapping; and
      the time interval is sufficiently long for the transient effect to substantially dissipate.
2. The treatment apparatus of embodiment 1 further comprising a second source of radiation coupled to the rotatable gantry system, the second source of radiation configured for at least one of imaging radiation or therapeutic radiation, wherein the second source of radiation has an energy level more than the first source of radiation.
3. The treatment apparatus of embodiment 1, wherein the radiation detector associated with the first source comprises a semiconductor material.
4. The treatment apparatus of embodiment 3, wherein the radiation detector comprises an energy-resolved photon-counting detector.
5. The treatment apparatus of embodiment 4, wherein the radiation detector is configured to discriminate energies of detected x-ray photons in the received radiation to generate the tomographic data.
6. The treatment apparatus of embodiment 1, wherein:
   the radiation detector comprises a semiconductor material;
   a bias voltage is provided to the semiconductor material when the radiation detector is receiving x-ray radiation; and
   the bias voltage is modified during the time interval between periodic emissions.
7. The treatment apparatus of embodiment 6, wherein modification of the applied bias includes at least one of voltage reduction, nullification, and reversal.
8. The treatment apparatus of embodiment 6, wherein the modification of the bias voltage is substantially applied to the interval between the periodic x-ray emission but can be initiated while the x-ray is still on and continue while the x-ray is restarted.
9. The treatment apparatus of embodiment 6, wherein the modification of the bias voltage facilitates dissipation of the transient effect.
10. The treatment apparatus of embodiment 2, wherein the semiconductor material comprises at least one of silicon, cadmium, tellurium, and zinc.
11. The treatment apparatus of embodiment 10, wherein the radiation detector comprises at least one of a cadmium zinc telluride (CdZnTe) detector, a silicon drift detector (SDD), a silicon PIN diode detector, and a cadmium telluride (CdTe) pixel detector.
12. The treatment apparatus of embodiment 1, wherein:
   the tomographic data includes images and is collected at an imaging frame rate; and
   the imaging frame rate is 1000 frames per second or less.
13. The treatment apparatus of embodiment 12, wherein the imaging frame rate is 700 frames per second or less.
14. The treatment apparatus of embodiment 2, wherein the first source of radiation comprises a kilo-electron volt peak photon energy (keV) up to 150 keV and the second source of radiation comprises a mega-electron volt peak photon energy (MeV) of 1 MeV or greater.
15. The treatment apparatus of embodiment 2, wherein the second source of radiation comprises a peak energy of 6 MeV.
16. The treatment apparatus of embodiment 1, wherein the radiation detector receives the radiation during a helical scan.
17. The treatment apparatus of embodiment 1, wherein the radiation detector receives the radiation during an axial scan.
18. A treatment apparatus, comprising:
   a first source of radiation configured to emit imaging x-ray radiation after pausing for a time interval between periodic emissions; and
   a radiation detector configured to receive x-ray radiation from the first radiation source and generate tomographic data from the received radiation, wherein:
      the radiation detector is subject to a transient effect caused by at least one of polarization and charge trapping;
      the time interval is sufficiently long for the transient effect to substantially dissipate;
      the radiation detector comprises a semiconductor material;
      a bias voltage is provided to the semiconductor material when the radiation detector is receiving x-ray radiation; and
      the bias voltage is modified during the time interval between periodic emissions to facilitate dissipation of the transient effect.
19. The treatment apparatus of embodiment 18 further comprising a second source of radiation, the second source of radiation configured for at least one of imaging radiation or therapeutic radiation, wherein the second source of radiation has an energy level more than the first source of radiation.
20. The treatment apparatus of embodiment 18, wherein the radiation detector comprises an energy-resolved photon-counting detector.
21. The treatment apparatus of embodiment 20, wherein the radiation detector is configured to analyze energies of detected x-ray photons in the received radiation to generate the tomographic data.
22. The treatment apparatus of embodiment 18, wherein:
   the tomographic data includes images and is collected at an imaging frame rate; and
   the imaging frame rate is 1000 frames per second or less.
23. The treatment apparatus of embodiment 22, wherein the imaging frame rate is 700 frames per second or less.
24. A treatment method, comprising:
   receiving, via a radiation detector, imaging x-ray radiation from a first radiation source, the first radiation source configured to emit the imaging x-ray radiation after pausing for a time interval between periodic emissions, wherein:
      the radiation detector is subject to a transient effect caused by at least one of polarization and charge trapping;
      the time interval is sufficiently long for the transient effect to substantially dissipate; and
      the radiation detector comprises a semiconductor material;
   providing a bias voltage to the semiconductor material when the radiation detector is receiving x-ray radiation from the first radiation source; and
   modifying the bias voltage during the time interval between periodic emissions to facilitate dissipation of the transient effect.
25. The method of embodiment 24 further comprising providing, via a second source of radiation, therapeutic radiation to a patient, wherein the second source of radiation has an energy level more than the first source of radiation.
26. The method of embodiment 25, wherein the first source of radiation comprises a kilo-electron volt peak photon energy (keV) up to 150 keV and the second source of radiation comprises a mega-electron volt peak photon energy (MeV) of 1 MeV or greater.
27. The method of embodiment 24, wherein the radiation detector comprises an energy-resolved photon-counting detector.
28. The method of embodiment 27, further comprising analyzing energies of detected x-ray photons in the received radiation to generate the tomographic data.
29. The method of embodiment 24, wherein the radiation detector comprises at least one of a cadmium zinc telluride (CdZnTe) detector, a silicon drift detector (SDD), a silicon PIN diode detector, and a cadmium telluride (CdTe) pixel detector.
30. The method of embodiment 24, wherein:
   the tomographic data includes images and is collected at an imaging frame rate; and
   the imaging frame rate is 1000 frames per second or less.
31. The method of embodiment 30, wherein the imaging frame rate is 700 frames per second or less.

While the embodiments discussed herein have been related to the systems and methods discussed above, these embodiments are intended to be exemplary and are not intended to limit the applicability of these embodiments to only those discussions set forth herein. While the present invention has been illustrated by the description of embodiments thereof, and while the embodiments have been described in some detail, it is not the intention of the applicant to restrict or in any way limit the scope of the appended claims to such detail. Additional advantages and modifications will readily appear to those skilled in the art. Therefore, the invention in its broader aspects is not limited to the specific details, representative apparatus and methods, and illustrative examples shown and described. Accordingly, departures may be made from such details without departing from the spirit or scope of the applicant's general inventive concept.

## Claims

1. A treatment apparatus, comprising:
a rotatable gantry system positioned at least partially around a patient support;
a first source of radiation coupled to the rotatable gantry system, the first source of radiation configured to emit imaging x-ray radiation after pausing for a time interval between periodic emissions; and
a radiation detector configured to receive x-ray radiation from the first radiation source and generate tomographic data from the received radiation, wherein:
the radiation detector is subject to a transient effect caused by at least one of polarization and charge trapping; and
the time interval is sufficiently long for the transient effect to substantially dissipate.

2. The treatment apparatus of claim 1 further comprising a second source of radiation coupled to the rotatable gantry system, the second source of radiation configured for at least one of imaging radiation or therapeutic radiation, wherein the second source of radiation has an energy level more than the first source of radiation.

3. The treatment apparatus of claim 1, wherein the radiation detector associated with the first source comprises a semiconductor material, in particular, wherein the radiation detector comprises an energy-resolved photon-counting detector.

4. The treatment apparatus of claim 3, wherein the radiation detector is configured to discriminate energies of detected x-ray photons in the received radiation to generate the tomographic data.

5. The treatment apparatus of claim 1, wherein:
the radiation detector comprises a semiconductor material;
a bias voltage is provided to the semiconductor material when the radiation detector is receiving x-ray radiation; and
the bias voltage is modified during the time interval between periodic emissions.

6. The treatment apparatus of claim 5, wherein modification of the applied bias includes at least one of voltage reduction, nullification, and reversal, and/or
wherein the modification of the bias voltage is substantially applied to the interval between the periodic x-ray emission but can be initiated while the x-ray is still on and continue while the x-ray is restarted, and/or
wherein the modification of the bias voltage facilitates dissipation of the transient effect.

7. The treatment apparatus of claim 2, wherein the semiconductor material comprises at least one of silicon, cadmium, tellurium, and zinc, in particular, wherein the radiation detector comprises at least one of a cadmium zinc telluride (CdZnTe) detector, a silicon drift detector (SDD), a silicon PIN diode detector, and a cadmium telluride (CdTe) pixel detector.

8. The treatment apparatus of claim 1, wherein:
the tomographic data includes images and is collected at an imaging frame rate; and
the imaging frame rate is 1000 frames per second or less, in particular, wherein the imaging frame rate is 700 frames per second or less.

9. The treatment apparatus of claim 2, wherein the first source of radiation comprises a kilo-electron volt peak photon energy (keV) up to 150 keV and the second source of radiation comprises a mega-electron volt peak photon energy (MeV) of 1 MeV or greater, and/or
wherein the second source of radiation comprises a peak energy of 6 MeV.

10. The treatment apparatus of claim 1, wherein the radiation detector receives the radiation during a helical scan and/or wherein the radiation detector receives the radiation during an axial scan.

11. A treatment method, comprising:
receiving, via a radiation detector, imaging x-ray radiation from a first radiation source, the first radiation source configured to emit the imaging x-ray radiation after pausing for a time interval between periodic emissions, wherein:
the radiation detector is subject to a transient effect caused by at least one of polarization and charge trapping;
the time interval is sufficiently long for the transient effect to substantially dissipate; and
the radiation detector comprises a semiconductor material;
providing a bias voltage to the semiconductor material when the radiation detector is receiving x-ray radiation from the first radiation source; and
modifying the bias voltage during the time interval between periodic emissions to facilitate dissipation of the transient effect.

12. The method of claim 11 further comprising providing, via a second source of radiation, therapeutic radiation to a patient, wherein the second source of radiation has an energy level more than the first source of radiation, in particular, wherein the first source of radiation comprises a kilo-electron volt peak photon energy (keV) up to 150 keV and the second source of radiation comprises a mega-electron volt peak photon energy (MeV) of 1 MeV or greater.

13. The method of claim 11, wherein the radiation detector comprises an energy-resolved photon-counting detector, in particular, further comprising analyzing energies of detected x-ray photons in the received radiation to generate the tomographic data.

14. The method of claim 11, wherein the radiation detector comprises at least one of a cadmium zinc telluride (CdZnTe) detector, a silicon drift detector (SDD), a silicon PIN diode detector, and a cadmium telluride (CdTe) pixel detector.

15. The method of claim 11, wherein:
the tomographic data includes images and is collected at an imaging frame rate; and
the imaging frame rate is 1000 frames per second or less, in particular, wherein the imaging frame rate is 700 frames per second or less.
